(19) Europäisches Patentamt
European Patent Office
Office européen des brevets

(11) Veröffentlichungsnummer: **0 370 383 A2**

(12) # EUROPÄISCHE PATENTANMELDUNG

(21) Anmeldenummer: **89121190.6**

(22) Anmeldetag: **16.11.89**

(51) Int. Cl.5: **C07K 5/02, C07C 335/06, A61K 37/64**

Claims for the following Contracting States: ES + GR.

(30) Priorität: **19.11.88 DE 3839128**

(43) Veröffentlichungstag der Anmeldung: **30.05.90 Patentblatt 90/22**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rüger, Wolfgang, Dr.**
**Parkstrasse 10**
**D-6233 Kelkheim(Taunus)(DE)**
Erfinder: **Urbach, Hansjörg, Dr.**
**Le Lavandoustrasse 41**
**D-6242 Kronberg/Taunus(DE)**
Erfinder: **Ruppert, Dieter, Dr.**
**Schreyerstrasse 30**
**D-6242 Kronberg/Taunus(DE)**
Erfinder: **Schölkens, Bernward, Prof. Dr.**
**Hölderlinstrasse 62**
**D-6233 Kelkheim(Taunus)(DE)**

(54) **Renin-hemmende Dipeptide, Verfahren zu ihrer Herstellung, diese enthaltende Mittel und deren Verwendung.**

(57) Die Erfindung betrifft Verbindungen der Formel I

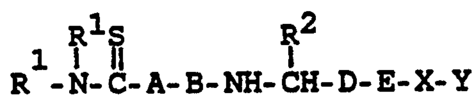

(I)

in welcher

A den Rest einer natürlichen oder unnatürlichen Aminosäure

B den Rest einer natürlichen oder unnatürlichen Aminosäure

D eine Gruppe aus der Reihe

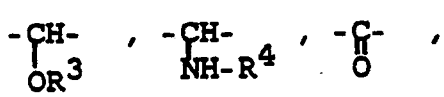

$-CH_2-SO_x-$ oder $-CH_2-NH-$

E abwesend ist oder eine Gruppe aus der Reihe $-(CH_2)_{1-3}-$, $-CHF-$, $-CF_2-$,

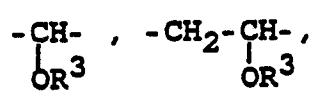

$-CH_2-SO_y-$,

EP 0 370 383 A2

$$-\underset{R^5}{\underset{|}{C}}H-,\quad -CH_2-\underset{R^5}{\underset{|}{C}}H-\quad -CH=\underset{R^6}{\underset{|}{C}}-$$

X abwesend ist oder eine Gruppe aus der Reihe
$-\underset{O}{\underset{\|}{C}}-$, $-(CF_2)_{1-4}-$, $-NH-\underset{O}{\underset{\|}{C}}-$ bedeuten
und
Y sowie $R^1$ bis $R^6$ wie in der Beschreibung angegeben definiert sind,
sowie Verfahren zu ihrer Herstellung, diese enthaltende Mittel und deren Verwendung.

# Renin-hemmende Dipeptide, Verfahren zu ihrer Herstellung, diese enthaltende Mittel und deren Verwendung

Aus den EP-A-172 346, EP-A-172 347, EP-A-189 203, EP-A-229 667 EP-A-230 266, EP-A-255 082, EP-A-273 893 und EP-A-274 259 sind Dipeptidderivate und deren Verwendung als Renin-Inhibitoren bekannt.

Es wurden neue Dipeptidderivate gefunden, die trotz der Abwesenheit eines bisher als essentiell betrachteten Strukturelementes überraschend in vitro und in vivo hochwirksam das Enzym Renin hemmen.

Die Erfindung betrifft Verbindungen der Formel I

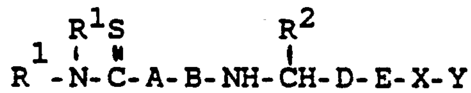

(I)

in welchen

$R^1$ unabhängig voneinander Wasserstoff, $(C_1$-$C_{10})$-Alkyl, das gegebenenfalls ein-oder zweifach ungesättigt ist und das gegebenenfalls durch bis zu 3 gleiche oder verschiedene Reste aus der Reihe Hydroxy, $(C_1$-$C_7)$-Alkoxy, $(C_1$-$C_7)$-Alkanoyloxy, Carboxy, $(C_1$-$C_7)$-Alkoxycarbonyl, Cl, Br, Amino, $(C_1$-$C_7)$-Alkylamino, Di-$(C_1$-$C_7)$-alkylamino, $(C_1$-$C_5)$-Alkoxycarbonylamino, $(C_7$ -$C_{15})$-Aralkoxycarbonylamino und 9-Fluorenylmethyloxycarbonylamino substituiert ist, $(C_3$-$C_8)$-Cycloalkyl, $(C_3$-$C_8)$-Cycloalkyl-$(C_1$-$C_6)$-alkyl, $(C_6$-$C_{14})$-Aryl, das gegebenfalls durch einen oder zwei gleiche oder verschiedene Reste aus der Reihe F, Cl, Br, J, Hydroxy, $(C_1$-$C_7)$-Alkoxy, $(C_1$-$C_7)$-Alkyl, $(C_1$-$C_7)$-Alkoxycarbonyl, Amino, gegebenenfalls mit bis zu 2 Halogen substituiertes Anilino und Trifluormethyl substituiert ist; $(C_6$-$C_{14})$-Aryl-$(C_1$-$C_6)$-alkyl, worin der Arylteil gegebenenenfalls durch einen oder zwei gleiche oder verschiedene Reste aus der Reihe F, Cl, Br, J, Hydroxy, $(C_1$-$C_7)$-Alkoxy, $(C_1$-$C_7)$-Alkyl, $(C_1$-$C_7)$-Alkoxycarbonyl, Amino, $(C_1$-$C_7)$-Alkylamino, Di-$(C_1$-$C_7)$-alkylamino, Carboxy, Carboxymethoxy, Amino-$(C_1$-$C_7)$-alkyl, $(C_1$-$C_7)$-Alkylamino-$(C_1$-$C_7)$alkyl, Di-$(C_1$-$C_7)$-alkylamino-$(C_1$-$C_7)$-alkyl, $(C_1$-$C_7)$-Alkoxycarbonylmethoxy, Carbamoyl, Sulfamoyl, $(C_1$-$C_7)$-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1 - 4 N-Atomen und/oder 1 S- oder O-Atom als Ringglieder steht, der gegebenenfalls durch einen, zwei oder drei gleiche oder verschiedene Reste aus der Reihe F, Cl, Br, Hydroxy, $(C_1$-$C_7)$-Alkoxy, $(C_1$-$C_7)$-Alkyl, $(C_1$-$C_7)$-Alkoxycarbonyl, Amino oder Trifluormethyl substituiert ist, bedeutet oder zusammen mit dem benachbarten Stickstoffatom und $R^1$ einen 3- bis 8-gliedrigen Monocyclus oder einen 7- bis 13-gliedrigen Bicyclus mit mindestens 1 C-Atom, 1 - 4 N-Atomen und/oder 1 S-oder O-Atom als Ringglieder, der gegebenenfalls durch ein, zwei oder drei gleiche oder verschiedene Reste aus der Reihe $(C_1$-$C_6)$-Alkyl, Hydroxy, Hydroxy-$(C_1$-$C_6)$-alkyl, $(C_1$-$C_6)$-Alkoxy, $(C_1$-$C_6)$-Alkoxy-$(C_1$-$C_6)$-alkyl, Carboxy, Carboxy-$(C_1$-$C_6)$-alkyl, $(C_1$-$C_6)$-Alkoxycarbonyl, $(C_1$-$C_6)$-Alkoxycarbonyl-$(C_1$-$C_6)$-alkyl, Halogen, Amino, Amino-$(C_1$-$C_6)$-alkyl, $(C_1$-$C_6)$-Alkylamino-$(C_1$-$C_6)$-alkyl, Di-$(C_1$-$C_6)$-alkylamino oder Di-$(C_1$-$C_6)$-alkylamino-$(C_1$-$C_6)$-alkyl substituiert sein kann, bildet;

A einen N-terminal mit -CS- und C-terminal mit B verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, Benzodioxol-5-ylalanin, N-methyl-histidin, 2-Amino-4-(3-thienyl)buttersäure, 3-(2-Thienyl)-serin, (Z)-Dehydrophenylalanin, (E)-Dehydrophenylalanin, (1,3-Dioxolan-2-yl)alanin, N-Pyrrolylalanin, (1-, 3- oder 4-Pyrazolyl)alanin, (4-Thiazolyl)alanin, (2-, 4- oder 5-Pyrimidyl)alanin, Cyclopentylglycin, tert. Butylglycin, Phenylserin, bedeutet, wobei die Aminogruppe gegebenenfalls jeweils durch $(C_1$-$C_6)$-Alkyl, Formyl, $(C_1$-$C_6)$-Alkoxycarbonyl oder Benzyloxycarbonyl, substituiert sein kann;

B einen N-terminal mit A und C-terminal mit -NH-CH-$R^2$-verknüpften Rest einer natürlichen oder unnatürlichen Aminosäure, wie für A definiert, wobei die Aminogruppe gegebenenfalls wie unter A beschrieben substituiert sein kann;

$R^2$ Wasserstoff, $(C_1$-$C_{10})$-Alkyl, $(C_4$-$C_7)$-Cycloalkyl, $(C_4$-$C_7)$-Cycloalkyl-$(C_1$-$C_4)$-alkyl, $(C_6$-$C_{14})$-Aryl, $(C_6$-

$C_{14}$)-Aryl-($C_1$-$C_4$)-alkyl oder Heterocyclyl-($C_1$-$C_4$)-alkyl bedeutet, wobei der Heterocyclus 4 - 7 Ringglieder, davon - oder 2 Schwefel- und/oder Sauerstoffatome, enthält;

D eine Gruppe aus der Reihe

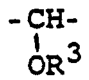 , 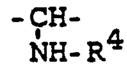 , 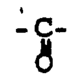 ,

$-CH_2-SO_x-$ oder $-CH_2-NH-$bedeutet, worin

$R^3$ Wasserstoff; ($C_1$-$C_{10}$)-Alkyl; ($C_1$-$C_6$)-Alkanoyl; ($C_6$-$C_9$)-Cycloalkanoyl; Phenyl, Phenyl-($C_1$-$C_4$)-alkyl, Benzoyl, die jeweils gegebenenfalls im Aromaten durch einen, Zwei oder drei Reste aus der Gruppe ($C_1$-$C_4$)-Alkyl, ($C_1$-$C_4$)-Alkoxy, Cl, F, Br, Nitro, Trifluormethyl oder ein Methylendioxy substituiert sind; ($C_1$-$C_{10}$)-Alkylaminothiocarbonyl, ($C_1$-$C_{10}$)-Alkylaminocarbonyl, wobei der ($C_1$-$C_{10}$)-Alkylrest jeweils wie unter $R^1$ beschrieben substituiert sein kann, ($C_1$-$C_6$)-Alkanoyloxy-($C_1$-$C_2$)-alkyl, ($C_1$-$C_6$)-Alcoxycarbonyloxy-($C_1$-$C_2$)-alkyl oder

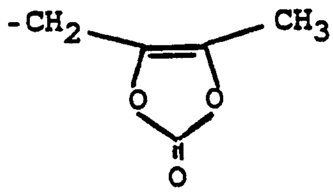

bedeutet,

$R^4$ Wasserstoff, ($C_1$-$C_6$)-Alkanoyl, ($C_5$-$C_8$)-Cycloalkanoyl, Phenylcarbonyl, Phenyl-($C_1$-$C_4$)-alkanoyl, N-($C_1$-$C_4$)-Alkyl-1,4-dihydronicotinoyl, einen Rest

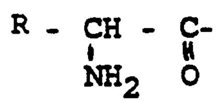 ,

worin R die Seitenkette einer natürlichen Aminosäure ist, ($C_1$-$C_6$)-Alkoxycarbonyl, ($C_5$-$C_8$)-Cycloalkyloxycarbonyl, Phenoxycarbonyl, ($C_1$-$C_6$)-Alkanoyloxy-($C_1$-$C_2$)-alkoxycarbonyl, Phenylcarbonyloxy-($C_1$-$C_2$)-alkoxycarbonyl, ($C_1$-$C_6$)-Alkanoyloxy-($C_1$-$C_2$)-alkyl, Phenylcarbonyloxy-($C_1$-$C_2$)-alkyl, ($C_1$-$C_6$)-Alkanoylaminomethyl, Phenylcarbonylaminomethyl oder ein gegebenenfalls substituiertes, über die 3-Position angeknüpftes Acrylsäurederivat, wobei in allen Resten $R^4$, die Phenyl enthalten, dieser Phenylrest gegebenenfalls durch ein, zwei oder drei Reste aus der Gruppe ($C_1$-$C_4$)-Alkyl, ($C_1$-$C_4$)-Alkoxy, Cl, Br, F, Nitro, Trifluormethyl oder ein Methylendioxy substituiert sein kann, bedeuten

x 0,1 oder 2 bedeutet

und

E abwesend ist oder eine Gruppe aus der Reihe $-(CH_2)_{1-3}-$, -CHF-, $-CF_2-$,

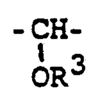 , 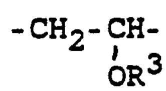 ,

$-CH_2-SO_y-$,

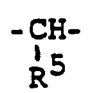 , 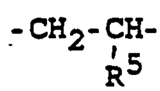 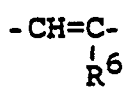

bedeutet,

worin

$R^3$ wie oben definiert ist,

$R^5$ die Seitenkette einer natürlichen Aminosäure, Hydroxy-($C_1$-$C_6$)-alkyl, ($C_1$-$C_6$)-Alkoxy oder ($C_2$-$C_6$)-Alkenyloxy bedeutet,

$R^6$ Wasserstoff oder ($C_1$-$C_6$)-Alkyl ist,

y 0,1 oder 2 bedeutet,

und

X abwesend ist oder eine Gruppe aus der Reihe

$-\underset{\underset{O}{\|}}{C}-$, $-(CF_2)_{1-4}-$ $-NH-\underset{\underset{O}{\|}}{C}-$ bedeutet

und

Y Wasserstoff, Fluor, ($C_1$-$C_6$)-Alkyl, ($C_5$-$C_8$)-Cycloalkyl, ($C_1$-$C_6$)-Alkoxy, ($C_5$-$C_8$)-Cycloalkoxy, Amino, ($C_1$-$C_6$)-Alkylamino, Di-($C_1$-$C_6$)-alkylamino, ($C_6$-$C_{14}$)-Aryl, ($C_6$-$C_{14}$)-Aryl-($C_1$-$C_6$)-alkyl, wobei jeweils der Arylrest unsubstituiert oder, wie oben unter $R^3$ definiert, mono-, di- oder trisubstituiert sein kann; Hydroxy, Azido, Azido-($C_1$-$C_6$)-alkyl, ($C_1$-$C_6$)-Alkylthio, ($C_1$-$C_6$)-Alkylsulfonyl, ($C_5$-$C_8$)-Cycloalkylthio, ($C_5$-$C_8$)-Cycloalkylsulfonyl, Heterocyclyl oder Heterocyclyl-($C_1$-$C_6$)-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist, bedeutet,

sowie deren physiologisch verträglichen Salze.

Die Chiralitätszentren in den Verbindungen der Formel I können die R-, S- oder R,S-Konfiguration aufweisen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

($C_6$-$C_{14}$)-Aryl ist beispielsweise Phenyl, Naphthyl, Biphenylyl oder Fluorenyl; bevorzug ist Phenyl. Entsprechendes gilt für davon abgeleitete Reste, wie z.B. Aryloxy, Aroyl, Aralkyl und Aralkyloxy. Unter Aralkyl versteht man einen mit ($C_1$-$C_6$)-Alkyl verknüpften unsubstituierten oder substituierten ($C_6$-$C_{14}$) Aryl Rest, wie z.B. Benzyl, α- und β-Naphthylmethyl, Halobenzyl und Alkoxybenzyl, wobei Aralkyl jedoch nicht auf die genannten Reste beschränkt ist.

Unter einem Rest eines 3- bis 8-gliedrigen monocyclischen oder 7- bis 13-gliedrigen bicyclischen Heterocyclus mit mindestens 1 C-Atom, 1-4 N-Atomen und/oder 1 S- oder O-Atom als Ringglieder werden Reste von Heteroaromaten verstanden, wie sie beispielsweise in Katritzky, Lagowski, Chemie der Heterocyclen, Berlin, Heidelberg 1968, Seite 3 - 5 definiert sind, sowie deren teilweise oder vollständig hydrierten Derivate. Der Heteroaromaten-Rest kann durch einen, zwei oder drei, vorzugsweise einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert sein. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für von Heteroaryl abgeleitete Reste, wie z.B. ganz oder teilweise hydriertes Heteroaryl, Heteroaryloxy, Heteroaryl und Heteroaryl-alkyl.

Die Aminosäuren A und B in Formel I sind durch eine Amidbindung miteinander verknüpft, es handelt sich um natürliche oder nichtnatürliche α-Aminosäuren der L-, D-oder D,L-Konfiguration, vorzugsweise der L-Konfiguration.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon-oder Sulfonsäuren wie z.B. Essigsäure, Citronensäure, Benzoesäure, Maleinsäure, Fumarsäure, Weinsäure und p-Toluolsulfonsäure.

Bevorzugt sind Verbindungen der Formel I, in welchen

$R^1$ unabhängig voneinander Wasserstoff- ($C_1$-$C_{10}$)-Alkyl das gegebenenfalls bis zu drei gleiche oder verschiedene Reste aus der Reihe Hydroxy, ($C_1$-$C_4$)-Alkoxy, ($C_1$-$C_4$)-Alkanoyloxy, Carboxy, ($C_1$-$C_4$)-Alkoxycarbonyl, Cl- Br- Amino, ($C_1$-$C_4$)-Alkylamino- Di-($C_1$-$C_4$)-alkylamino oder ($C_1$-$C_4$)-Alkoxycarbonylamino substituiert ist, ($C_5$-$C_7$)-Cycloalkyl, ($C_6$-$C_{14}$)-Aryl, das gegebenenfalls durch einen oder zwei gleiche oder verschiedene Reste aus der Reihe F, Cl, Br, Hydroxy, ($C_1$-$C_4$)-Alkoxy, ($C_1$-$C_4$)-Alkyl, ($C_1$-$C_4$)-Al-

koxycarbonyl, Amino und Trifluormethyl substituiert ist, ($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, Hydroxy, ($C_1$-$C_4$)-Alkoxy, ($C_1$-$C_4$)-Alkyl, ($C_1$-$C_4$)-Alkoxycarbonyl, Amino, ($C_1$-$C_4$)-Alkylamino, Di-($C_1$-$C_4$) alkylamino, Carboxy, Carboxymethoxy, Amino-($C_1$-$C_4$)-alkyl, ($C_1$-$C_4$)-Alkylamino-($C_1$-$C_4$)-alkyl, Di-($C_1$-$C_4$)-alkylamino-($C_1$-$C_4$)-alkyl, ($C_1$-$C_4$)-Alkoxycarbonylmethoxy, Carbamoyl, Sulfamoyl, ($C_1$-$C_4$)-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1 - 4 N-Atomen und/oder 1 S- oder O-Atomen als Ringglieder steht, der gegebenenfalls durch einen, zwei oder drei gleiche oder verschiedene Reste aus der Reihe F, Cl, Br, Hydroxy, ($C_1$-$C_4$)-Alkoxy, ($C_1$-$C_4$)-Alkyl, ($C_1$-$C_4$)-Alkoxycarbonyl, Amino oder Trifluormethyl substituiert ist, bedeutet oder zusammen mit dem benachbarten Stickstoffatom und $R^1$ einen 3- bis 8-gliedrigen Monocyclus oder einen 7- bis 13-gliedrigen Bicyclus mit mindestens 1 C-Atom, 1 - 4 N-Atomen und/oder 1 S-oder O-Atom als Ringglieder, der gegebenfalls durch ein, zwei oder drei gleiche oder verschiedene Reste aus der Reihe ($C_1$-$C_6$)-Alkyl, Hydroxy, Hydroxy-($C_1$-$C_6$)-alkyl, ($C_1$-$C_6$)-Alkoxy, ($C_1$-$C_6$)-Alkoxy-($C_1$-$C_6$)-alkyl, Carboxy, Carboxy-($C_1$-$C_6$)-alkyl, ($C_1$-$C_6$) Alkoxycarbonyl, ($C_1$-$C_6$)-Alkoxycarbonyl-($C_1$-$C_6$)-alkyl, Halogen, Amino, Amino-($C_1$-$C_6$)-alkyl, ($C_1$-$C_6$)-Alkylamino-($C_1$-$C_6$)-alkyl, Di-($C_1$-$C_6$)-alkylamino oder Di-($C_1$-$C_6$)-alkylamino-($C_1$-$C_6$)-alkyl substituiert sein kann, bildet;

A einen N-terminal mit -CS- und C-terminal mit B verknüpften Rest einer natürlichen oder unnatürlichen Aminosäure aus der Reihe Phenylalanin, Histidin, Tyrosin, Tryptophan, ß-2-Thienylalanin, ß-3-Thienylalanin, ß-2-Furylalanin, ß-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyldopa, 2-Amino-4-(2-thienyl)-buttersäure, Benzodioxol-5-ylalanin, N-methyl-histidin, 2-Amino-4-(3-thienyl)buttersäure, 3-(2-Thienyl)-serin,(Z)-Dehydrophenylalanin oder (E)-Dehydrophenylalanin, bedeutet;

B einen N-terminal mit A und C-terminal mit -NH-CH-$R^2$-verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, Benzodioxol-5-ylalanin, N-Methyl-histidin, 2-Amino-4-(3-thienyl)-buttersäure, 3-(2-Thienyl)-serin,(Z)-Dehydrophenylalanin, (E)-Dehydrophenylalanin, (1,3-Dioxolan-2-yl)alanin, N-Pyrrolylalanin, (1-, 3- oder 4-Pyrazolyl)alanin, (4-Thiazolyl-alanin, (2-, 4- oder 5-Pyrimidyl)alanin, Cyclopentylglycin, tert. Butylglycin oder Phenylserin, bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl ($C_6$-$C_{14}$)-Aryl-($C_1$-$C_4$)-alkyl oder Heterocyclyl-($C_1$-$C_4$)-alkyl bedeutet, wobei der Heterocyclus 4 - 7 Ringglieder, davon 1 oder 2 Schwefel-und/oder Sauerstoffatome, besitzt,

und

D eine Gruppe aus der Reihe

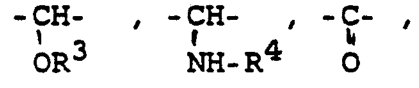

-$CH_2$-$SO_x$- oder -$CH_2$-NH-bedeutet, worin

$R^3$ Wasserstoff,

$R^4$ Wasserstoff,

x 0, 1 oder 2 bedeutet

und

E abwesend ist oder eine Gruppe aus der Reihe -$(CH_2)_{1-3}$-, -CHF-, -$CF_2$-,

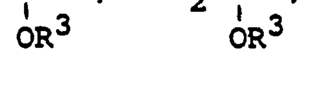

$-CH_2-SO_y-$,

$$\underset{R^5}{\underset{|}{-CH-}} \quad , \quad -CH_2-\underset{R^5}{\underset{|}{CH-}} \quad , \quad -CH=\underset{R^6}{\underset{|}{C-}}$$

bedeutet
worin
$R^3$ Wasserstoff ist,
$R^5$ die Seitenkette einer natürlichen Aminosäure, Hydroxy-($C_1$-$C_6$)-alkyl, ($C_1$-$C_6$)-Alkoxy oder ($C_2$-$C_6$)-Alkenyloxy bedeutet,
$R^6$ Wasserstoff oder ($C_1$-$C_6$)-Alkyl ist,
y 0, 1 oder 2 bedeutet
und
X abwesend ist oder eine Gruppe aus der Reihe
$-\underset{\overset{\|}{O}}{C}-$, $-(CF_2)_{1-4}-$, $-NH-\underset{\overset{\|}{O}}{C}-$ bedeutet
und
Y Wasserstoff, Fluor, ($C_1$-$C_6$)-Alkyl, ($C_5$-$C_8$)-Cycloalkyl, ($C_1$-$C_6$)-Alkoxy, ($C_5$-$C_8$)-Cycloalkoxy, Amino, ($C_1$-$C_6$)-Alkylamino, Di-($C_1$-$C_6$)-alkylamino, ($C_6$-$C_{14}$)-Aryl, ($C_6$-$C_{14}$)-Aryl-($C_1$-$C_6$)-alkyl, wobei jeweils der Arylrest unsubstituiert oder, wie oben unter R $^3$ definiert, mono-, di oder trisubstituiert sein kann, Hydroxy, Azido, Azido-($C_1$-$C_6$)- alkyl, ($C_1$-$C_6$)-Alkylthio, ($C_1$-$C_6$)-Alkylsulfonyl, ($C_5$-$C_8$)-Cycloalkylthio, ($C_5$-$C_8$)-Cycloalkylsulfonyl, Heterocyclyl oder Heterocyclyl-($C_1$ $C_6$)-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist bedeutet,
sowie deren physiologisch verträgliche Salze.

Besonders bevorzugt sind Verbindungen der Formel I, in welchen
$R^1$ unabhängig voneinander Wasserstoff, ($C_1$-$C_{10}$)-Alkyl, das gegebenenfalls durch bis zu drei gleiche oder verschiedene Reste aus der Reihe Hydroxy, Carboxy, Amino, ($C_1$-$C_4$)-Alkylamino- Di-($C_1$-$C_4$)-alkylamino oder ($C_1$-$C_4$)-Alkoxycarbonylamino substituiert ist, Phenyl, das gegebenenfalls durch ein oder zwei Reste aus der Reihe F, Cl, Hydroxy, Amino oder Trifluormethyl substituiert ist, Phenyl-($C_1$-$C_4$)-alkyl worin der Phenylteil gegebenenfalls durch einen oder zwei gleiche oder verschiedene Reste aus der Reihe F, Cl, Hydroxy, Amino, ($C_1$-$C_4$)-Alkylamino, Di-($C_1$-$C_4$)-alkylamino, Carboxy, Amino-($C_1$-$C_4$)-alkyl, ($C_1$-$C_4$)-Alkylamino-($C_1$-$C_4$)-alkyl, Di-($C_1$-$C_4$)-alkylamino-($C_1$-$C_4$)-alkyl substituiert ist, bedeutet, oder zusammen mit dem benachbarten Stickstoffatom und $R^1$ einen 3- bis 8-gliedrigen Monocyclus oder einen 7- bis 13-gliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe ($C_1$-$C_6$)-Alkyl, Hydroxy, Hydroxy-($C_1$-$C_6$)-alkyl, ($C_1$-$C_6$)-Alkoxy-($C_1$-$C_6$)-alkyl, Carboxy, Carboxy-($C_1$-$C_6$)-alkyl, ($C_1$-$C_6$)-Alkoxycarbonyl, ($C_1$-$C_6$)-Alkoxycarbonyl-($C_1$-$C_6$)-alkyl, Halogen, Amino, Amino-($C_1$-$C_6$)-alkyl, ($C_1$-$C_6$)-Alkylamino-($C_1$-$C_6$)-alkyl, Di-($C_1$-$C_6$)alkylamino oder Di-($C_1$-$C_6$)-alkylamino-($C_1$-$C_6$)-alkyl substituiert sein kann, bildet
A einen N-terminal mit -CS- und C-terminal mit B verknüpften Rest einer Aminosäure aus der Reihe Phenylalanin, Tyrosin, ß-2-Thienylalanin, ß-3-Thienylalanin, 4-Chlorphenylalanin, O-Methyltyrosin, 1-Naphthylalanin, 2-Fluorphenylalanin, 3-Fluorphenylalanin, 4-Fluorphenylalanin bedeutet,
B einen N terminal mit A und C-terminal mit $-NH-CHR^2$-verknüpften Rest einer Aminosäure aus der Reihe Phenylalanin, Histidin, Leucin, ß-2-Thienylalanin, ß-3-Thienylalanin, Lysin, Norvalin, 2-Fluorphenylalanin, 3-Fluorphenylalanin, 4-Fluorphenylalanin, Norleucin, S-Methylcystein, (1,3-Dioxolan-2-yl)alanin, (1-, 3- oder 4-Pyrazolyl)alanin 4-Thiazolylalanin- (2-, 4- oder 5-Pyrimidyl)alanin bedeutet
$R^2$ Isobutyl, Cyclohexylmethyl, Benzyl oder (1,3-Dithiolan-2-yl)methyl bedeutet,
D eine Gruppe

$$\underset{OR^3}{\underset{|}{-CH-}}$$

bedeutet und $R^3$ Wasserstoff ist,
E abwesend ist oder eine Gruppe aus der Reihe $-(CH_2)_{1-3}-$,

$$-\underset{OR^3}{\underset{|}{CH}}- \ , \ -CH_2-\underset{OR^3}{\underset{|}{CH}}- \ , \ -\underset{R^5}{\underset{|}{CH}}-$$

bedeutet,
worin
$R^3$ Wasserstoff ist und
$R^5$ die Seitenkette einer natürlichen Aminosäure bedeutet
X abwesend ist oder eine Gruppe aus der Reihe
$-\underset{O}{\underset{\|}{C}}-$, $-NH-\underset{O}{\underset{\|}{C}}-$ bedeutet
und
Y Wasserstoff, $(C_1-C_6)$-Alkyl, $(C_5-C_8)$-Cycloalkyl, Amino, $(C_1-C_6)$-Alkylamino, Di-$(C_1-C_6)$-alkylamino, $(C_6-C_{14})$-Aryl, $(C_6-C_{14})$-Aryl-$(C_1-C_6)$-alkyl, wobei jeweils der Arylrest unsubstituiert oder, wie oben unter $R^3$ definiert, mono-, di- oder trisubstituiert sein kann, Hydroxy, Azido, $(C_1-C_6)$-Alkylthio, $(C_1-C_6)$-Alkylsulfonyl, $(C_5-C_8)$-Cycloalkylthio, $(C_5-C_8)$-Cycloalkylsulfonyl, Heterocyclyl oder Heterocyclyl-$(C_1-C_6)$-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist bedeutet,
sowie deren physiologisch verträgliche Salze.

Die Verbindung betrifft weiterhin ein Verfahren zur Herstellung von Verbindungen der allgemeinen Formel I, das dadurch gekennzeichnet ist, daß man

a) eine Verbindung der allgemeinen Formel II

$$H-A-B-NH-\overset{R^2}{\overset{|}{CH}}-D-E-X-Y \qquad (II)$$

in welcher A, B, D, E, X, Y und $R^2$ die gleiche Bedeutung wie in der allgemeinen Formel I (Seite 1) haben, mit einem Isothiocyanat der allgemeinen Formel III
$R^1-N=C=S$ (III)
worin $R^1$ die gleiche Bedeutung wie in der allgemeinen Formel I hat,
in einem geeigneten organischen Lösungsmittel wie z. B. Benzol, Toluol, Chlorbenzol, einem aliphatischen Kohlenwasserstoff, einem aliphatischen Chlorkohlenwasserstoff, Diethylether, Tetrahydrofuran, Dioxan, Aceton, Acetonitril, Dimethylformamid, Dimethylsulfoxid, oder aber unter Verzicht auf ein Lösungsmittel jeweils wahlweise mit oder ohne Zusatz einer Lewis-Säure oder einer Lewis-Base als Katalysator, wie z. B. eines tertiären Amins, vorzugsweise Triethylamin, 1,4-Diazabicyclo[2.2.2]octan, Cyclohexyldimethylamin, Benzyldimethylamin, 4-Methylmorpholin, Tetramethylguanidin oder 4-Dimethylaminopyridin, bei einer Temperatur zwischen -80 °C und der Siedetemperatur des Lösungsmittels, vorzugsweise zwischen -30 °C und +80 °C, umsetzt, gegebenenfalls zuvor empfindliche funktionelle Gruppen wie Hydroxy- oder Aminogruppen mit einer Schutzgruppe versieht und diese Schutzgruppe am Ende wieder entfernt,
oder

b) eine Verbindung der allgemeinen Formel IV mit freier Carboxylgruppe

$$R^1-\overset{R^1}{\overset{|}{N}}-\overset{S}{\overset{\|}{C}}-A-OH \qquad (IV)$$

worin $R^1$ und A die gleiche Bedeutung wie in Formel I haben, oder ein reaktives Derivat hiervon mit dem entsprechenden Fragment mit einer freien Aminogruppe der allgemeinen Formel V

$$H-B-NH-\overset{R^2}{\overset{|}{CH}}-D-E-X-Y \qquad (V)$$

worin B, D, E, X, Y und $R^2$ die gleiche Bedeutung wie in Formel I besitzen, kuppelt und gegebenenfalls zum Schutz weiterer funktioneller Gruppen (eine) temporär eingeführte Schutzgruppe(n) abspaltet,

oder

c) eine Verbindung der allgemeinen Formel VI mit freier Carboxylgruppe

$$R^1-\overset{R^1}{\overset{|}{N}}-\overset{S}{\overset{\|}{C}}-A-B-OH \qquad (VI)$$

worin $R^1$, A und B die gleiche Bedeutung wie in Formel I haben, oder ein reaktives Derivat hiervon mit dem entsprechenden Fragment mit einer freien Aminogruppe der allgemeinen Formel VII

$$H_2N-\overset{R^2}{\overset{|}{CH}}-D-E-X-Y \qquad (VII)$$

worin $R^2$, D, E, X und Y die gleiche Bedeutung wie in Formel I haben- kuppelt und gegebenenfalls zum Schutz weiterer funktioneller Gruppen (eine) temporär eingeführte Schutzgruppe(n) abspaltet, und die so erhaltene Verbindung in ihr physiologisch verträgliches Salz überführt.

Methoden, die bei den Verfahrensvarianten b) und c) zur Herstellung einer Amidbindung geeignet sind, werden z.B. in Houben-Weyl, Methoden der organischen Chemie, Band 15/2; Bodanszky et al., Peptide Synthesis, 2nd ed. (Wiley & Sons, New York 1976) oder Gross, Meienhofer, The Peptides. Analysis, Synthesis, Biology (Academic Press, New York 1979) beschrieben. Vorzugsweise werden die folgenden Methoden herangezogen: Aktivestermethode mit N-Hydroxy-succinimid als Esterkomponente, Kupplung mit einem Carbodiimid wie Dicyclohexylcarbodiimid oder mit Propanphosphonsäureanhydrid - und die Gemischt-Anhydrid-Methode mit Pivaloylchlorid.

Die zur Herstellung von Verbindungen der Formel I erforderlichen Vor- und Nachoperationen wie Einführung und Abspaltung von Schutzgruppen sind literaturbekannt und sind z. B. in T.W. Greene, "Protective Groups in Organic Synthesis" beschrieben. Salze von Verbindungen der Formel I mit salzbildenden Gruppen werden in an sich bekannter Weise hergestellt, indem man z. B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Säuren A oder B anfallen, können in an sich bekannter Weise durch fraktionierte Kristallisation oder durch Chromatographie getrennt werden.

Verbindungen der allgemeinen Formel II werden erhalten aus Verbindungen der allgemeinen Formel VIII

$$SG-A-B-NH-\overset{R^2}{\overset{|}{CH}}-D-E-X-Y \qquad (VIII)$$

worin A, B, D, E, X, Y und $R^2$ die gleiche Bedeutung wie in Formel I haben und SG eine Amino-Schutzgruppe bedeutet wie z. B. eine tert. Butyloxycarbonyl- oder eine Benzyloxycarbonyl-Schutzgruppedurch Abspaltung dieser Schutzgruppe unter den üblichen Bedingungen, z. B. durch saure Hydrolyse oder durch Hydrogenolyse.

Die Isothiocyanate der allgemeinen Formel III sind literaturbekannt bzw. auf analogem Wege wie die literaturbekannten Verbindungen aus käuflichen Vorstufen erhältlich.

Verbindungen der allgemeinen Formel IV, lassen sich in literaturbekannter Weise aus den Isothiocyanaten der allgemeinen Formel III und den Aminosäuren H-A-OH in freier oder OH-geschützter Form herstellen.

Verbindungen der allgemeinen Formel V werden erhalten durch Kupplung von Verbindungen der Formel VII mit geschützten Aminosäurederivaten SG-B-OH, worin SG die gleiche Bedeutung wie in Formel VIII und B die gleiche Bedeutung wie in Formel I besitzt, unter Bedingungen wie sie unter der Verfahrensvariante b) beschrieben sind, gefolgt von einer Abspaltung der Schutzgruppe SG.

Verbindungen der allgemeinen Formel VI sind erhältlich durch Umsetzung von Dipeptiden H-A-B-OH in freier oder OH-geschützter Form mit Isothiocyanaten der allgemeinen Formel III oder durch Kupplung von Verbindungen der allgemeinen Formel IV mit Aminosäuren H-B-OH, gegebenenfalls in OH-geschützter Form, gefolgt von einer Abspaltung der gegebenenfalls benutzten Schutzgruppe.

Verbindungen der allgemeinen Formel VII sind die sogenannten Zentralbausteine bereits bekannter Renininhibitoren, deren Herstellung je nach Definition der Reste $R^2$, D, E, X und Y stark variiert und in

zahlreichen Patentanmeldungen sowie Publikationen beschrieben ist. Beispielsweise und stellvertretend für weitere Literaturstellen seien hier genannt: EP 255 082, EP 230 266, EP 229 667, EP 258 183, DOS 3 729 729; J. Chem. Soc. Perkin Trans. I 1987, 1177, J. Med. Chem. 30 1617 (1987); J. Org. Chem. 53 869 (1988); usw.

Verbindungen der allgemeinen Formel VIII sind erhältlich durch Kupplungen von geschützten Aminosäurederivaten SG-A-OH an Verbindungen der allgemeinen Formel V oder durch Kupplung von geschützten Dipeptiden SG-A-B-OH an Verbindungen der allgemeinen Formel VII.

Die erfindungsgemäßen Verbindungen der Formel I weisen enzymhemmende Eigenschaften auf; insbesondere hemmen sie die Wirkung des natürlichen Enzyms Renin. Dies ist insofern überraschend und unerwartet, als alle bekannten Renin-Inhibitoren (für eine neuere Übersicht siehe z. B. W. Greenlee, Pharmac. Res. 4, 364 (1987)) an der $P^4$-Position (d.h. derjenigen Position des Renin-Inhibitors, die der -CS-Funktion der Verbindungen der Formel I äquivalent ist) eine Carbonylgruppe oder eine Sulfongruppe tragen. Sie besitzen somit in dieser Position ein Sauerstoffatom, das als Wasserstoffbrückenakzeptor fungieren kann und für die Wechselwirkung mit dem Enzym potentiell von großer Bedeutung ist. Es ist daher überraschend, daß der Ersatz des Sauerstoffs durch ein Schwefelatom mit seinem größeren Volumen und seier geringen Tendenz zur Ausbildung von Wasserstoffbrücken ebenfalls zu hochwirksamen Renin-Inhibitoren führt, die die Wirkung der Sauerstoff-analogen Verbindungen sogar noch übertreffen.

Renin ist ein proteolytisches Enzym aus der Klasse der Aspartyl-Proteasen, welches als Folge verschiedener Stimuli (Volumendepletion, Natriummangel, ß-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

## 1. Testprinzip

Z.B. Humanplasma, welches sowohl Renin als auch Angiotensinogen enthält, wird bei 37 °C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

## 2. Gewinnung des Plasmas

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4 °C, 15 min; wiederholen, falls erforderlich) wird das Plasma vorsichtig abpipettiert und in geeigneten Portionen bei -30 °C eingefroren. Für den Test werden nur Plasmen mit ausreichend hoher Reninaktivität verwendet. Plasmen mit niedriger Reninaktivität werden durch eine Kältebehandlung (-4 °C, 3 Tage) aktiviert (Prorenin → Renin).

## 3. Durchführung des Tests

Angiotensin I wird mit dem Renin-Maia$^{(R)}$-Kit (Serono Diagnostics S.A., Coinsins, Schweiz) bestimmt. Die Inkubation des Plasmas wird nach der dort angegebenen Anleitung durchgeführt:
Inkubationsansatz:
1000 µl Plasma (bei 0-4 °C aufgetaut)

100 µl Phosphatpuffer (pH 7,4) Zusatz von $10^{-4}$ M Ramiprilat)
10 µl PMSFf-Lösung
10 µl 0,1 % Genapol PFIC
12 µl DMSO bzw. Testpräparat

Die Testpräparate werden i.a. $10^{-2}$ M in 100 % Dimethylsulfoxid (DMSO)- gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37° C) gestellt. Aus einem zusätzlichen Ansatz ohne Inhibitor werden ohne weitere Inkubation insgesamt 6 Proben (Jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25° C für die Angiotensin I-Bestimmung aufbewahrt (Mittelwert aus drei Einzelproben).

**Angiotensin I-Radioimmunoassay (RIA)**

Es wird exakt die Gebrauchsanweisung des RIA-Kits (Renin-Maia(R)-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von aillen Meßwerten abgezogen. Die Plasma-Renin-Aktivität (PRA) wird als ng Ang I/ml x Stunde angegeben. PRA-werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel I zeigen in dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten, wie zum Beispiel Rhesus-Affen, herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1 - 50 mg/kg. Die in der vorliegenden Erfindung beschriebenen Verbindungen der allgemeinen Formel I können als Antihypertensiva sowie zur Behandlung der Herzinsuffizienz verwendet werden.

Die Erfindung betrifft daher auch die Verwendung von Verbindungen der Formel I als Heilmittel und pharmazeutische Präparate, die diese Verbindungen enthalten. Bevorzugt ist die Anwendung bei Primaten, insbesondere beim Menschen.

Pharmazeutische Präparate enthalten eine wirksame Menge des Wirkstoffs der Formel I zusammen mit einem anorganischen oder organischen pharmazeutisch verwendbaren Trägerstoff.

Die Anwendung kann intranasal, intravenös, subkutan, peroral oder intraduodenal erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Mischt-, Granuliert-oder Dragierverfahren hergestellt.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol,

Propandiol oder Glycerin, daneben auch Zuckerlösungen wie Glucose-oder Mannitlösungen, oder auch eine Mischung aus den verschiedenen genannten Lösungsmitteln.

Verzeichnis der verwendeten Abkürzungen:

AcOH Essigsäure
Boc tert.-Butoxycarbonyl
DCI Desorption Chemical Ionisation
DNP 2,4-Dinitrophenyl
DMF Dimethylformamid
EI Electron Impact
FAB Fast atom bombardment
M Molekularpeak
MeOH Methanol
MPLC Mitteldruck-Säulenchromatographie
MS Massenspektrum
R.T. Raumtemperatur
Schmp. Schmelzpunkt

Die sonstigen für Aminosäuren verwendeten Abkürzungen entsprechen dem in der Peptidchemie üblichen Drei-Buchstaben-Code, wie er z. B. in Eur. J. Biochem. 138, 9-37 (1984) beschrieben ist. Falls nicht ausdrücklich anders angegeben, handelt es sich immer um Aminosäuren der L-Konfiguration.

Die nachstehenden Beispiele dienen der Erläuterung der vorliegenden Erfindung, ohne diese darauf zu beschränken.

**Beispiel 1:**

1-Cyclohexyl-6-(2-pyridyl)-2S-[N-(tert. butylamino-thiocarbonyl-Phe-His)-amino]-3S-hexanol

1a) H-His(DNP)-OH

Zu einer Lösung von 0,42 g (1 mmol) Boc-His(DNP)-OH in 5 ml Dimethoxyethan tropft man bei 0-5° C 4 ml HCl-gesättigtes Dimethoxyethan und rührt 1 Stunde bei 0-5° C und 3 Stunden bei Raumtemperatur nach. Die Reaktionslösung wird im Vakuum eingeengt und noch zweimal mit Toluol abgeraucht.
Ausbeute: 0.5 g der Titelverbindung als Hydrochlorid. Rf (Methylenchlorid/MeOH/AcOH/Wasser 70:30:1:1) = 0,16.

1b) Boc-Phe-His(DNP)-OH

Zu 0,5 g (1 mmol) H-His(DNP)-OH-hydrochlorid in 12,5 ml einer 0,25 N Natriumhydrogencarbonatlösung gibt man bei Raumtemperatur 0,362 g (1 mmol) Boc-Phe-hydroxysuccinimidester, gelöst in 5 ml Ethanol und 5 ml THF. Das Reaktionsgemisch wird drei Tage bei Raumtemperatur gerührt. Dann setzt man 0,83 g Citronensäure zu, wobei die Titelverbindung ölig ausfällt. Das Produkt wird mit Methylenchlorid extrahiert, die organische Phase über Natriumsulfat getrocknet, eingeengt und der Rückstand mit wenig Essigester versetzt, die Titelverbindung durch Zusatz von Diisopropylether ausgefällt und abgesaugt.
Ausbeute: 0,47 g (83 %); Rf (Methylenchlorid/MeOH 7:3) = 0,43; MS (FAB) = 569 (M+1).

1c) 2S-Amino-1-cyclohexyl-6-(2-pyridyl)-3S-hexanol

Zu 214 mg (0.513 mmol) 3-Boc-4S-cyclohexylmethyl-2,2-dimethyl-5-(3-(2-pyridyl)-propyl)-oxazolidin in 10 ml Dimethoxymethan tropft man im Eisbad 5 ml HCl-gesättigtes Dimethoxyethan und rührt eine Stunde bei 0° C und 5 Stunden bei Raumtemperatur nach. Die Reaktionslösung wird im Vakuum eingeengt und noch zweimal mit Toluol abgeraucht.

Ausbeute: 179 mg der Titelverbindung als Dihydrochlorid.

1d) 1-Cyclohexyl-6-(2-pyridyl)-2S-(N-(Boc-Phe-His(DNP))-amino)-3S-hexanol

1-5 g (3,18 mmol) 2S-Amino-1-cyclohexyl-6-(2-pyridyl)-3S-hexanol-dihydrochlorid (Beispiel 1c) und 1,8 g (3,18 mmol) Boc-Phe-His(DNP)-OH (Beispiel 1b) werden in 15 ml absolutem Dimethylformamid gelöst. Dazu gibt man 0,59 g (6,36 mmol) 1-Hydroxybenzotriazol-hydrat und kühlt im Eisbad auf ca. 4° C ab. Bei dieser Temperatur gibt man nacheinander 2,44 ml (19,1 mmol) N-Ethylmorpholin und 0,65 g (3,18 mmol) Dicyclohexylcarbodiimid zu und rührt 1 Stunde im Eisbad sowie 7 Stunden bei Raumtemperatur nach. Nach Stehen über Nacht werden erneut 0,9 g (1,59 mmol) Boc-Phe-His(DNP)-OH, 0,28 g (3-18 mmol) 1-Hydroxybenzotriazol-hydrat und 0-32 g (1-58 mmol) Dicyclohexylcarbodiimid hinzugegeben und weitere 10 Stunden bei Raumtemperatur gerührt. Man saugt vom Niederschlag ab, engt das Filtrat im Vakuum ein, löst den Rückstand in Essigester und wäscht mit gesättigter Natriumhydrogencarbonatlösung, Wasser und gesättigter Natriumchloridlösung, trocknet über Natriumsulfat, engt ein und reinigt das Rohprodukt (3,8 g) durch Mitteldruck-Säulenchromatographie an Kieselgel (Methylenchlorid/MeOH 98:2, 95:5, 9:1).
Man erhält 1,8 g (68 %) der Titelverbindung.
Rf (Methylenchlorid/MeOH 9:1) = 0,46; MS (FAB) = 827 (M+1).

1e) 1-Cyclohexyl-6-(2-pyridyl)-2S-(N-(Phe-His(DNP))-amino)-3S-hexanol

72 mg 1-Cyclohexyl-6-(2-pyridyl)-2S-(N-(Boc-Phe-His(DNP))-amino 3S-hexanol werden bei 0-5° C mit 2 ml eiskalter Trifluoressigsäure übergossen und zwei Stunden bei dieser Temperatur nachgerührt. Die Reaktionslösung wird im Vakuum eingeengt und zweimal mit Toluol abgeraucht.
Ausbeute: 83 mg der Titelverbindung als Bis(trifluoracetat).

1f) 1-Cyclohexyl-6-(2-pyridyl)-2S-[N-(tert. butylaminothiocarbonyl-Phe-His(DNP)-amino]-3S-hexanol

Zu einer Lösung von 88 mg (0.092 mmol) 1-Cyclohexyl-6-(2-pyridyl)-2S-[N-(Phe-His(DNP))-amino]-3S-hexanol (aus Beispiel 1e) in 2 ml absolutem DMF gibt man 0,06 ml (0,42 mmol) Triethylamin und 0,061 ml (0,46 mmol) tert. Butylisothiocyanat, rührt 5 Stunden bei Raumtemperatur und läßt übers Wochenende stehen. Dann wird 1 Stunde bei 40° C gerührt, noch einmal die gleiche Menge tert. Butylisothiocyanat zugesetzt, weitere 2 Stunden bei 40° C und 9 Stunden bei 60° C gerührt. Die Reaktionslösung wird auf Eiswasser gegeben, mit Essigester extrahiert und die vereinigten Extrakte mit gesättigter Natriumbicarbonatlösung, Wasser und gesättigter Natriumchloridlösung gewaschen, über Natriumsulfat getrocknet, eingeengt und das Rohprodukt (86 mg) durch MPLC an Kieselgel (Methylenchlorid/MeOH 95:5, 9:1) gereinigt.
Ausbeute: 39,3 mg (51 %) der Titelverbindung.
Rf (Methylenchlorid/MeOH 9:1) = 0,39; MS (FAB) = 842 (M+1).

1g) 1-Cyclohexyl-6-(2-pyridyl)-2S-[N-(tert.-butylaminothiocarbonyl-Phe-His)-amino]-3S-hexanol

39 mg (0,046 mmol) 1-Cyclohexyl-6-(2-pyridyl)-2S-[N-(tert.-butylaminothiocarbonyl-Phe-His(DNP))-amino]-3S-hexanol (aus Beispiel 1f) werden in 5 ml Acetonitril mit 0,1 ml (1 mmol) Thiophenol versetzt und 4 Stunden bei Raumtemperatur gerührt. Die Reaktionslösung wird im Vakuum eingeengt und das Rohprodukt durch MPLC an Kieselgel (Methylenchlorid/MeOH 95:5, 9:1) gereinigte.
Man erhält 24,3 mg (78 %) der Titelverbindung.
Rf (Methylenchlorid/MeOH 9:1) = 0,14; MS (FAB) = 676 (M+1).

## Beispiel 2

1-Cyclohexyl-6-(2-pyridyl)-2S-[N-(tert. butylaminothiocarbonyl-Phe-Nva)-amino]-3S-hexanol

2a) Boc-Phe-Nva-OMe

15,0 g (0,056 mol) Boc-Phe-OH und 9,4 g (0,056 mol) Nva-OMe-hydrochlorid werden in 250 ml absolutem Methylenchlorid gelöst. Dazu tropft man unter Eiskühlung zunächst 38,6 ml (0.28 mol) absolutes Triethylamin und dann 36,4 ml Propanphosphonsäureanhydrid (50 % in Methylenchlorid). Die Reaktionslösung wird 3 Stunden bei Raumtemperatur nachgerührt und über Nacht stehengelassen. Zur Hydrolyse gibt man auf Eiswasser, rührt 2 Stunden kräftig nach, trennt die organische Phase ab und wäscht sie mit 10 %iger Citronensäurelösung, gesättigter Natriumhydrogencarbonatlösung und Wasser, trocknet über Natriumsulfat und engt ein.
Ausbeute 20,8 g (98 %) der Titelverbindung.
Rf (Methylenchlorid/MeOH 9 : 1) = 0.69; MS (DCI) = 378 (M+1)

2b) Boc-Phe-Nva-OH

20,1 g (0,053 mol) Boc-Phe-Nva-OMe (Beispiel 2a) werden in 30 ml Wasser und 30 ml Dioxan suspendiert. Bei Raumtemperatur werden 2,5 g (0,106 mol) Lithiumhydroxid eingetragen und 3 Stunden bei Raumtemperatur nachgerührt. Die Reaktionslösung wird mit 10 %iger Natriumhydrogensulfatlösung angesäuert und das Produkt abgesaugt und mit Diisopropylether verrührt.
Ausbeute: 19,1 g (98 %), MS (DCI) = 364 (M+1)

2c) 1-Cyclohexyl-6-(2-pyridyl)-2S-(N-(Boc-Phe-Nva)-amino)-3S-hexanol

Aus 1,7 g (3,36 mmol) 2S-Amino-1-cyclohexyl-6-(2-pyridyl)-3S-hexanol-dihydrochlorid (Beispiel 1c) und 1,22 g 3,36 mmol) Boc-Phe-Nva-OH (Beispiel 2b) erhält man analog dem in Beispiel 1d) angegebenen Verfahren 0,8 g der Titelverbindung.
Rf (Methylenchlorid/MeOH 9:1) = 0,53; MS (FAB) = 623 (M+1)

2d) 1-Cyclohexyl-6-(2-pyridyl)-2S-(N-(Phe-Nva)-amino)-3S-hexanol

Aus 97 mg 1-Cyclohexyl-6-(2-pyridyl)-2S-(N-(Boc-Phe-Nva)-amino)-3S-hexanol (Beispiel 2c) erhält man analog dem in Beispiel 1c) angegebenen Verfahren 92 mg der Titelverbindung als Dihydrochlorid.

2e) 1-Cyclohexyl-6-(2-pyridyl)-2S-[N-(tert. butylaminothiocarbonyl-Phe-Nva)-amino]-3S-hexanol

101 mg (0,17 mmol) 1-Cyclohexyl-6-(2-pyridyl)-2S-[N-(Phe-Nva)-amino]-3S-hexanol (aus Beispiel 2d) werden in 4 ml absolutem Dimethylformamid gelöst und bei Raumtemperatur nacheinander mit 104 µl (0,73 mmol) frisch destilliertem Triethylamin und 22,5 µl (0,17 mmol) Tert. butylisothiocyanat versetzt. Nach Rühren über Nacht werden erneut 10 µl (0,076 mmol) Tert. butylisothiocyanat hinzugefügt, weitere 8 Stunden bei Raumtemperatur gerührt, das Lösungsmittel im Hochvakuum abdestilliert, der Rückstand in Essigester aufgenommen, mit gesättigter Natriumhydrogencarbonatlösung, Wasser und gesättigter Natriumchloridlösung gewaschen, über Natriumsulfat getrocknet, eingeengt und das Rohprodukt (129 mg) durch MPLC and Kieselgel (Methylenchlorid/Methanol 98:2; 95:5) gereinigt.
Man erhält 53,5 mg (49 %) der Titelverbindung.
Rf (Methylenchlorid/MeOH 9:1) = 0,43; MS (FAB) = 638 (M+1).

**Beispiel 3**

1-Cyclohexyl-6-(2-pyridyl)-2S-[N-(tert. butylaminothiocarbonyl-Phe-Nva)-amino]-3S-(tert. butylaminothiocarbonyloxy)-hexan

95 mg (0,16 mmol) 1-Cyclohexyl-6-(2-pyridyl)-2S-[N-(Phe-Nva)-amino]-3S-hexanol werden wie unter Beispiel 2e) beschrieben mit 0,104 ml (0,73 mmol) Triethylamin und 0,106 ml (0,8 mmol) Tert. butylisothiocyanat 10 Stunden lang bei Raumtemperatur zur Reaktion gebracht und analog aufgearbeitet.
Man erhält 16 mg (13 %) der Titelverbindung.
Rf (Methylenchlorid/MeOH 9:1) = 0,81;
MS (FAB) = 753 (M + 1).

Unter Verwendung geeigneter Ausgangsmaterialien und unter Verwendung der in den Beispielen 1-3 beschriebenen Verfahren wird auch folgende Verbindung erhalten:

**Beispiel 4**

1-Cyclohexyl-6-(2-pyridyl)-2S-[N-(tert. butylaminothiocarbonyl-Phe-Nva)-amino]-3R-hexanol

Rf (Methylenchlorid/MeOH 9:1) = 0,46; MS (FAB) = 638 (M + 1).

Beispiel 5

1-Cyclohexyl-6-(2-pyridyl)-2S-[N-(morpholinothiocarbonyl-Phe-Nva)-amino]-3S-hexanol

MS(FAB) = 652(M + 1)

Beispiel 6

1-Cyclohexyl-6-(2-pyridyl)-2S-[N-(morpholinothiocarbonyl-Phe-His)-amino]3S-hexanol

MS(FAB) = 690(M + 1)

**Ansprüche**

1. Verbindung der Formel I

$$R^1-\overset{\overset{R^1}{|}}{N}-\overset{\overset{S}{\|}}{C}-A-B-NH-\overset{\overset{R^2}{|}}{CH}-D-E-X-Y \qquad (I)$$

in welcher
$R^1$ unabhängig voneinander Wasserstoff, $(C_1-C_{10})$-Alkyl, das gegebenenfalls ein- oder zweifach ungesättigt ist und das gegebenenfalls durch bis zu 3 gleiche oder verschiedene Reste aus der Reihe Hydroxy, $(C_1-C_7)$-Alkoxy, $(C_1-C_7)$-Alkanoyloxy, Carboxy, $(C_1-C_7)$-Alkoxycarbonyl, Cl, Br, Amino, $(C_1-C_7)$-Alkylamino, Di-$(C_1-C_7)$-alkylamino, $(C_1-C_5)$-Alkoxycarbonylamino, $(C_7-C_{15})$-Aralkoxycarbonylamino und 9-Fluorenylmethyloxycarbonylamino substituiert ist, $(C_3-C_8)$-Cycloalkyl, $(C_3-C_8)$-Cycloalkyl-$(C_1-C_6)$-alkyl, $(C_6-C_{14})$-Aryl, das gegebenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino gegebenenfalls mit bis zu 2 Halogen substituiertes Anilino und Trifluormethyl substituiert ist; $(C_6-C_{14})$-Aryl-$(C_1-C_6)$-alkyl, worin der Arylteil gegebenenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1 - 4 N-Atomen und/oder 1 S- oder O-Atom als Ringglieder steht, der gegebenenfalls durch einen, zwei oder drei gleiche oder verschiedene Reste aus der Reihe F, Cl, Br, Hydroxy, $(C_1-C_7)$-Alkoxy, $(C_1-C_7)$-Alkyl, $(C_1-C_7)$-Al-

koxycarbonyl, Amino oder Trifluormethyl substituiert ist, bedeutet oder zusammen mit dem benachbarten Stickstoffatom und $R^1$ einen 3-bis 8-gliedrigen Monocyclus oder einen 7- bis 13-gliedrigen Bicyclus mit mindestens 1 C-Atom, 1 - 4 N-Atomen und/oder 1 S-oder O-Atom als Ringglieder, der gegebenenfalls durch ein, zwei oder drei gleiche oder verschiedene Reste aus der Reihe ($C_1$-$C_6$)-Alkyl, Hydroxy, Hydroxy-($C_1$-$C_6$)-alkyl, ($C_1$-$C_6$)-Alkoxy, ($C_1$-$C_6$)-Alkoxy-($C_1$-$C_6$)-alkyl, Carboxy, Carboxy-($C_1$-$C_6$)-alkyl, ($C_1$-$C_6$)-Alkoxycarbonyl, ($C_1$-$C_6$)-Alkoxycarbonyl-($C_1$-$C_6$)-alkyl, Halogen, Amino, Amino-($C_1$-$C_6$)-alkyl, ($C_1$-$C_6$)-Alkylamino-($C_1$-$C_6$)-alkyl, Di-($C_1$-$C_6$)-alkylamino oder Di-($C_1$-$C_6$)-alkylamino-($C_1$-$C_6$)-alkyl substituiert sein kann, bildet;

A einen N-terminal mit -CS- und C-terminal mit B verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, Benzodioxol-5-ylalanin, N-Methyl-histidin, 2-Amino-4-(3-thienyl)buttersäure, 3-(2 Thienyl)-serin, (Z)-Dehydrophenylalanin, (E)-Dehydrophenylalanin, (1,3-Dioxolan-2-yl)alanin, N-Pyrrolylalanin, (1-, 3- oder 4-Pyrazolyl)alanin, (4-Thiazolyl)alanin, (2-, 4 oder 5-Pyrimidyl)alanin, Cyclopentylglycin, tert. Butylglycin, Phenylserin, bedeutet, wobei die Aminogruppe gegebenenfalls jeweils durch ($C_1$-$C_6$)-Alkyl, Formyl, ($C_1$-$C_6$)-Alkoxycarbonyl oder Benzyloxycarbonyl, substituiert sein kann;

B einen N-terminal mit A und C-terminal mit -NH CH-$R^2$-verknüpften Rest einer natürlichen oder unnatürlichen Aminosäure, wie für A definiert, wobei die Aminogruppe gegebenenfalls wie unter A beschrieben substituiert sein kann;

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl, ($C_6$-$C_{14}$)-Aryl-($C_1$-$C_4$)-alkyl oder Heterocyclyl-($C_1$-$C_4$)-alkyl bedeutet, wobei der Heterocyclus 4 - 7 Ringglieder, davon 1 oder 2 Schwefel- und/oder Sauerstoffatome, enthält;

D eine Gruppe aus der Reihe

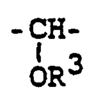 , 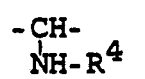 , 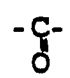 ,

$-CH_2-SO_x-$ oder $-CH_2-NH-$ bedeutet, worin

$R^3$ Wasserstoff; ($C_1$-$C_{10}$)-Alkyl; ($C_1$-$C_6$)-Alkanoyl; ($C_6$-$C_9$)-Cycloalkanoyl; Phenyl, Phenyl-($C_1$-$C_4$)-alkyl, Benzoyl, die jeweils gegebenenfalls im Aromaten durch einen, zwei oder drei Reste aus der Gruppe ($C_1$-$C_4$)-Alkyl, ($C_1$-$C_4$)-Alkoxy, Cl, F, Br, Nitro, Trifluormethyl oder ein Methylendioxy substituiert sind; ($C_1$-$C_{10}$)-Alkylaminothiocarbonyl, ($C_1$-$C_{10}$)-Alkylaminocarbonyl, wobei der ($C_1$-$C_{10}$)-Alkylrest jeweils wie unter $R^1$ beschrieben substituiert sein kann, ($C_1$-$C_6$)-Alkanoyloxy-($C_1$-$C_2$)-alkyl, ($C_1$-$C_6$)-Alcoxycarbonyloxy-($C_1$-$C_2$)-alkyl oder

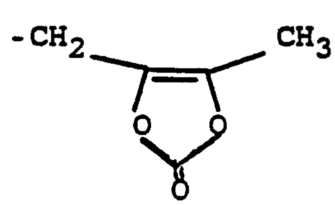

bedeutet,

$R^4$ Wasserstoff, ($C_1$-$C_6$)-Alkanoyl, ($C_5$-$C_8$)-Cycloalkanoyl, Phenylcarbonyl, Phenyl-($C_1$-$C_4$)-alkanoyl, N-($C_1$-$C_4$)-Alkyl-1,4-dihydronicotinoyl, einen Rest

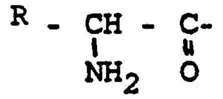 ,

worin R die Seitenkette einer natürlichen Aminosäure ist, $(C_1-C_6)$-Alkoxycarbonyl, $(C_5-C_8)$-Cycloalkyloxycarbonyl, Phenoxycarbonyl, $(C_1-C_6)$-Alkanoyloxy-$(C_1-C_2)$-alkoxycarbonyl, Phenylcarbonyloxy-$(C_1-C_2)$-alkoxycarbonyl, $(C_1-C_6)$-Alkanoyloxy-$(C_1-C_2)$-alkyl, Phenylcarbonyloxy-$(C_1-C_2)$-alkyl, $(C_1-C_6)$-Alkanoylaminomethyl, Phenylcarbonylaminomethyl oder ein gegebenenfalls substituiertes, über die 3-Position angeknüpftes Acrylsäurederivat, wobei in allen Resten $R^4$, die Phenyl enthalten, dieser Phenylrest gegebenenfalls durch ein, zwei oder drei Reste aus der Gruppe $(C_1-C_4)$-Alkyl, $(C_1-C_4)$-Alkoxy, Cl, Br, F, Nitro, Trifluormethyl oder ein Methylendioxy substituiert sein kann, bedeuten

x  0,1 oder 2 bedeutet

und

E  abwesend ist oder eine Gruppe aus der Reihe $-(CH_2)_{1-3}-$, -CHF-, $-CF_2-$,

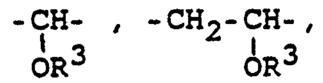

$-CH_2-SO_y-$,

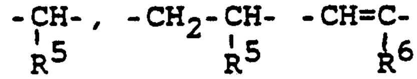

bedeutet,

worin

$R^3$ wie oben definiert ist,

$R^5$ die Seitenkette einer natürlichen Aminosäure, Hydroxy-$(C_1-C_6)$-alkyl, $(C_1-C_6)$-Alkoxy oder $(C_2-C_6)$-Alkenyloxy bedeutet,

$R^6$ Wasserstoff oder $(C_1-C_6)$-Alkyl ist,

y 0,1 oder 2 bedeutet,

und

X  abwesend ist oder eine Gruppe aus der Reihe

- C -, $-(CF_2)_{1-4}-$, -NH- C - bedeutet
  ‖                    ‖
  O                    O

und

Y  Wasserstoff, Fluor, $(C_1-C_6)$-Alkyl, $(C_5-C_8)$-Cycloalkyl, $(C_1-C_6)$-Alkoxy, $(C_5-C_8)$-Cycloalkoxy, Amino, $(C_1-C_6)$-Alkylamino, Di-$(C_1-C_6)$-alkylamino, $(C_6-C_{14})$-Aryl, $(C_6-C_{14})$-Aryl-$(C_1-C_6)$-alkyl, wobei jeweils der Arylrest unsubstituiert oder, wie oben unter $R^3$ definiert, mono-, di- oder trisubstituiert sein kann; Hydroxy, Azido, Azido-$(C_1-C_6)$-alkyl, $(C_1-C_6)$-Alkylthio, $(C_1-C_6)$-Alkylsulfonyl, $(C_5-C_8)$-Cycloalkylthio, $(C_5-C_8)$-Cycloalkylsulfonyl, Heterocyclyl oder Heterocyclyl-$(C_1-C_6)$-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist, bedeutet,

sowie deren physiologisch verträglichen Salze.

2. Verbindung der Formel I gemäß Anspruch 1- dadurch gekennzeichnet, daß

$R^1$  unabhängig voneinander Wasserstoff, $(C_1-C_{10})$-Alkyl- das gegebenenfalls bis zu drei gleiche oder verschiedene Reste aus der Reihe Hydroxy, $(C_1-C_4)$-Alkoxy, $(C_1-C_4)$-Alkanoyloxy, Carboxy, $(C_1-C_4)$-Alkoxycarbonyl, Cl, Br, Amino, $(C_1-C_4)$-Alkylamino, Di-$(C_1-C_4)$-alkylamino oder $(C_1-C_4)$-Alkoxycarbonylamino substituiert ist, $(C_5-C_7)$-Cycloalkyl, $(C_6-C_{14})$-Aryl, das gegebenenfalls durch einen oder zwei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino und Trifluormethyl substituiert ist, $(C_6-C_{14})$-Aryl-$(C_1-C_6)$-alkyl, worin der Arylteil gegebenenfalls durch einen oder zwei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino, $(C_1-C_4)$-Alkylamino, Di-$(C_1-C_4)$-alkylamino, Carboxy, Carboxymethoxy, Amino-$(C_1-C_4)$-alkyl, $(C_1-C_4)$-Alkylamino-$(C_1-C_4)$-alkyl, Di-$(C_1-C_4)$-alkylamino-$(C_1-C_4)$-alkyl, $(C_1-C_4)$-Alkoxycarbonylmethoxy, Carbamoyl, Sulfamoyl, $(C_1-C_4)$-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1 - 4 N-Atomen und/oder 1 S- oder O-Atomen als Ringglieder steht, der gegebenenfalls durch einen, zwei oder drei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino oder Trifluormethyl substituiert ist, bedeutet oder zusammen mit dem benachbarten Stickstoffatom und $R^1$ einen

3- bis 8-gliedrigen Monocyclus oder einen 7- bis 13-gliedrigen Bicyclus mit mindestens 1 C-Atom, 1 - 4 N-Atomen und/oder 1 S-oder O-Atom als Ringglieder, der gegebenfalls durch ein, zwei oder drei gleiche oder verschiedene Reste aus der Reihe ($C_1$-$C_6$)-Alkyl, Hydroxy, Hydroxy-($C_1$-$C_6$)-alkyl, ($C_1$-$C_6$)-Alkoxy, ($C_1$-$C_6$)-Alkoxy-($C_1$-$C_6$)-alkyl, Carboxy, Carboxy-($C_1$-$C_6$)-alkyl, ($C_1$-$C_6$)-Alkoxycarbonyl, ($C_1$-$C_6$)-Alkoxycarbonyl-($C_1$-$C_6$)-alkyl- Halogen Amino, Amino-($C_1$-$C_6$)-alkyl, ($C_1$-$C_6$)-Alkylamino-($C_1$-$C_6$)-alkyl, Di-($C_1$-$C_6$)-alkylamino oder Di-($C_1$-$C_6$)-alkylamino-($C_1$-$C_6$)-alkyl substituiert sein kann, bildet;

A einen N-terminal mit -CS- und C-terminal mit B verknüpften Rest einer natürlichen oder unnatürlichen Aminosäure aus der Reihe Phenylalanin, Histidin, Tyrosin, Tryptophan, ß-2-Thienylalanin, ß-3-Thienylalanin, ß-2-Furylalanin, ß-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyldopa, 2-Amino-4-(2-thienyl)-buttersäure, Benzodioxol-5-ylalanin, N-Methyl-histidin, 2-Amino-4-(3-thienyl)buttersäure, 3-(2-Thienyl)-serin,(Z)-Dehydrophenylalanin oder (E)-Dehydrophenylalanin, bedeutet;

B einen N-terminal mit A und C-terminal mit -NH-CH-$R^2$-verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, Benzodioxol-5-ylalanin, N-Methyl-histidin, 2-Amino-4-(3-thienyl)-buttersäure, 3-(2-Thienyl)-serin,(Z)-Dehydrophenylalanin, (E)-Dehydrophenylalanin, (1,3-Dioxolan-2-yl)alanin, N-Pyrrolylalanin, (1-, 3- oder 4-Pyrazolyl)alanin, (4-Thiazolyl)alanin, (2-, 4- oder 5-Pyrimidyl)alanin, Cyclopentylglycin, tert. Butylglycin oder Phenylserin, bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl, ($C_6$-$C_{14}$)-Aryl-($C_1$-$C_4$)-alkyl oder Heterocyclyl-($C_1$-$C_4$)-alkyl bedeutet, wobei der Heterocyclus 4 - 7 Ringglieder, davon 1 oder 2 Schwefel-und/oder Sauerstoffatome, besitzt,

und

D eine Gruppe aus der Reihe

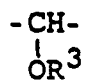 , 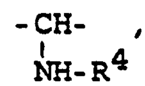 , 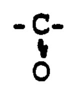 ,

$-CH_2-SO_x-$ oder $-CH_2-NH-$bedeutet, worin

$R^3$ Wasserstoff,

$R^4$ Wasserstoff,

x 0, 1 oder 2 bedeutet

und

E abwesend ist oder eine Gruppe aus der Reihe $-(CH_2)_{1-3}-$, -CHF-, $-CF_2-$,

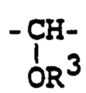 , 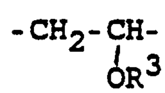 ,

$-CH_2-SO_y-$,

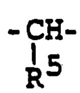 , 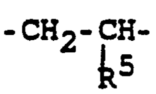 , 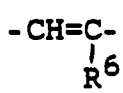

bedeutet

worin

$R^3$ Wasserstoff ist,

$R^5$ die Seitenkette einer natürlichen Aminosäure, Hydroxy-($C_1$-$C_6$)-alkyl, ($C_1$-$C_6$)-Alkoxy oder ($C_2$-$C_6$)-Alkenyloxy bedeutet,
$R^6$ Wasserstoff oder ($C_1$-$C_6$)-Alkyl ist, y 0, 1 oder 2 bedeutet
und
X abwesend ist oder eine Gruppe aus der Reihe
$-\underset{\overset{\|}{O}}{C}-$, $-(CF_2)_{1-4}-$, $-NH-\underset{\overset{\|}{O}}{C}-$ bedeutet
und
Y Wasserstoff- Fluor, ($C_1$-$C_6$)-Alkyl, ($C_5$-$C_8$)-Cycloalkyl, ($C_1$-$C_6$)-Alkoxy, ($C_5$-$C_8$)-Cycloalkoxy, Amino, ($C_1$-$C_6$)-Alkylamino, Di-($C_1$-$C_6$)-alkylamino ($C_6$-$C_{14}$)-Aryl, ($C_6$-$C_{14}$)-Aryl-($C_1$-$C_6$)-alkyl, wobei jeweils der Arylrest unsubstituiert oder, wie oben unter $R^3$ definiert, mono-, di oder trisubstituiert sein kann, Hydroxy, Azido, Azido-($C_1$-$C_6$)-alkyl, ($C_1$-$C_6$)-Alkylthio, ($C_1$-$C_6$)-Alkylsulfonyl, ($C_5$-$C_8$)-Cycloalkylthio, ($C_5$-$C_8$)-Cycloalkylsulfonyl, Heterocyclyl oder Heterocyclyl-($C_1$-$C_6$)-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist bedeutet,
sowie deren physiologisch verträgliche Salze.

3. Verbindung der Formel I, gemäß einem oder mehreren der Ansprüche 1 bis 2, dadurch gekennzeichnet, daß
$R^1$ unabhängig voneinander Wasserstoff, ($C_1$-$C_{10}$)-Alkyl, das gegebenenfalls durch bis zu drei gleiche oder verschiedene Reste aus der Reihe Hydroxy, Carboxy, Amino, ($C_1$-$C_4$)-Alkylamino, Di($C_1$-$C_4$)-alkylamino oder ($C_1$-$C_4$)-Alkoxycarbonylamino substituiert ist, Phenyl, das gegebenenfalls durch ein oder zwei Reste aus der Reihe F, Cl Hydroxy, Amino oder Trifluormethyl substituiert ist, Phenyl-($C_1$-$C_4$)-alkyl, worin der Phenylteil gegebenenfalls durch einen oder zwei gleiche oder verschiedene Reste aus der Reihe F, Cl, Hydroxy, Amino, ($C_1$-$C_4$)-Alkylamino, Di-($C_1$-$C_4$)-alkylamino, Carboxy, Amino-($C_1$-$C_4$)-alkyl, (Ci-$C_4$)-Alkylamino-($C_1$-$C_4$)-alkyl, Di-($C_1$-$C_4$)-alkylamino-($C_1$-$C_4$)-alkyl substituiert ist, bedeutet, oder zusammen mit dem benachbarten Stickstoffatom und $R^1$ einen 3- bis 8-gliedrigen Monocyclus oder einen 7- bis 13-gliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe ($C_1$-$C_6$)-Alkyl, Hydroxy, Hydroxy-($C_1$-$C_6$)-alkyl, ($C_1$-$C_6$)-Alkoxy-($C_1$-$C_6$)-alkyl, Carboxy, Carboxy-($C_1$-$C_6$)-alkyl, ($C_1$-$C_6$)-Alkoxycarbonyl, ($C_1$-$C_6$)-Alkoxycarbonyl-($C_1$-$C_6$)-alkyl, Halogen, Amino, Amino-($C_1$-$C_6$)-alkyl, ($C_1$-$C_6$)-Alkylamino-($C_1$-$C_6$)-alkyl, Di-($C_1$-$C_6$)alkylamino oder Di-($C_1$-$C_6$)-alkylamino-($C_1$-$C_6$)-alkyl substituiert sein kann, bildet
A einen N-terminal mit -CS- und C-terminal mit B verknüpften Rest einer Aminosäure aus der Reihe Phenylalanin, Tyrosin, ß-2-Thienylalanin, ß-3-Thienylalanin, 4-Chlorphenylalanin, O-Methyltyrosin, 1-Naphthylalanin, 2-Fluorphenylalanin, 3-Fluorphenylalanin, 4-Fluorphenylalanin bedeutet,
B einen N-terminal mit A und C-terminal mit -NH-CHR²-verknüpften Rest einer Aminosäure aus der Reihe Phenylalanin, Histidin, Leucin, ß-2-Thienylalanin, ß-3-Thienylalanin, Lysin, Norvalin, 2-Fluorphenylalanin, 3-Fluorphenylalanin, 4-Pluorphenylalanin, Norleucin, S-Methylcystein, (1,3-Dioxolan-2-yl)alanin, (1-, 3-oder 4-Pyrazolyl)alanin, 4-Thiazolylalanin, (2-, 4- oder 5-Pyrimidyl)alanin, bedeutet,
$R^2$ Isobutyl, Cyclohexylmethyl, Benzyl oder (1,3-Dithiolan-2-yl)methyl bedeutet,
D eine Gruppe

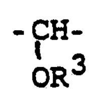

bedeutet und $R^3$ Wasserstoff ist,
E abwesend ist oder eine Gruppe aus der Reihe $-(CH_2)_{1-3}-$,

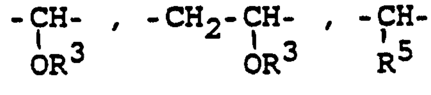

bedeutet,
worin
$R^3$ Wasserstoff ist und
$R^5$ die Seitenkette einer natürlichen Aminosäure bedeutet

X abwesend ist oder eine Gruppe aus der Reihe
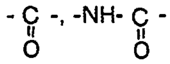 bedeutet
und

Y Wasserstoff, ($C_1$-$C_6$)-Alkyl, ($C_5$-$C_8$)-Cycloalkyl, Amino, ($C_1$-$C_6$)-Alkylamino, Di-($C_1$-$C_6$)-alkylamino, ($C_6$-$C_{14}$)-Aryl, ($C_6$-$C_{14}$)-Aryl-($C_1$-$C_6$)-alkyl, wobei jeweils der Arylrest unsubstituiert oder, wie oben unter $R^3$ definiert, mono-, di- oder trisubstituiert sein kann, Hydroxy, Azido, ($C_1$-$C_6$)-Alkylthio, ($C_1$-$C_6$)-Alkylsulfonyl, ($C_5$-$C_8$)-Cycloalkylthio, ($C_5$-$C_8$)-Cycloalkylsulfonyl, Heterocyclyl oder Heterocyclyl-($C_1$-$C_6$)-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist bedeutet,
sowie deren physiologisch verträgliche Salze.

4. Verfahren zur Herstellung einer Verbindung der allgemeinen Formel I, gemäß einem oder mehreren der Ansprüche 1 bis 3, dadurch gekennzeichnet, daß man

a) eine Verbindung der allgemeinen Formel II

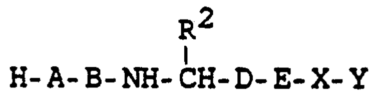 (II)

in welcher A, B, D, E, X, Y und $R^2$ die gleiche Bedeutung wie in der allgemeinen Formel I haben, mit einem Isothiocyanat der allgemeinen Formel III

$R^1$-N = C = S (III)

worin $R^1$ die gleiche Bedeutung wie in der allgemeinen Formel I hat,
in einem geeigneten organischen Lösungsmittel wie z. B. Benzol, Toluol, Chlorbenzol, einem aliphatischen Kohlenwasserstoff, einem aliphatischen Chlorkohlenwasserstoff, Diethylether, Tetrahydrofuran, Dioxan, Aceton, Acetonitril, Dimethylformamid, Dimethylsulfoxid, oder aber unter Verzicht auf ein Lösungsmittel jeweils wahlweise mit oder ohne Zusatz einer Lewis-Säure oder einer Lewis-Base als Katalysator, wie z. B. eines tertiären Amins, vorzugsweise Triethylamin, 1,4-Diazabicyclo[2.2.2]octan, Cyclohexyldimethylamin, Benzyldimethylamin, 4-Methylmorpholin, Tetramethylguanidin oder 4-Dimethylaminopyridin, bei einer Temperatur zwischen -80° C und der Siedetemperatur des Lösungsmittels, vorzugsweise zwischen -30° C und -80° C, umsetzt, gegebenenfalls zuvor empfindliche funktionelle Gruppen wie Hydroxy- oder Aminogruppen mit einer Schutzgruppe versieht und diese Schutzgruppe am Ende wieder entfernt,
oder

b) eine Verbindung der allgemeinen Formel IV mit freier Carboxylgruppe

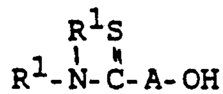 (IV)

worin $R^1$ und A die gleiche Bedeutung wie in Formel I haben, oder ein reaktives Derivat hiervon mit dem entsprechenden Fragment mit einer freien Aminogruppe der allgemeinen Formel V

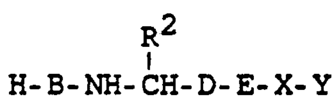 (V)

worin B, D, E, X, Y und $R^2$ die gleiche Bedeutung wie in Formel I besitzen, kuppelt und gegebenenfalls zum Schutz weiterer funktioneller Gruppen (eine) temporär eingeführte Schutzgruppe(n) abspaltet,
oder

c) eine Verbindung der allgemeinen Formel VI mit freier Carboxylgruppe

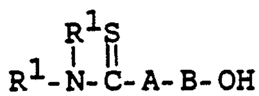 (VI)

worin $R^1$, A und B die gleiche Bedeutung wie in Formel I haben, oder ein reaktives Derivat hiervon mit dem

entsprechenden Fragment mit einer freien Aminogruppe der allgemeinen Formel VII

$$H_2N-\overset{R^2}{\underset{}{\overset{|}{C}}}H-D-E-X-Y \qquad (VII)$$

worin $R^2$, D, E, X und Y die gleiche Bedeutung wie in Formel I haben, kuppelt und gegebenenfalls zum Schutz weiterer funktioneller Gruppen (eine) temporär eingeführte Schutzgruppe(n) abspaltet, und die so erhaltene Verbindung in ihr physiologisch verträgliches Salz überführt.

5. Verwendung einer Verbindung der Formel I gemäß einem oder mehreren der Ansprüche 1 bis 3 als Heilmittel.

6. Verwendung einer Verbindung der Formel I gemäß einem oder mehreren der Ansprüche 1 bis 3 zur Behandlung des Bluthochdrucks.

7. Pharmazeutische Mittel enthaltend eine Verbindung der Formel I gemäß einem oder mehreren der Ansprüche 1 bis 3.

Patentansprüche für folgende Vertragsstaaten: ES; GR

1. Verfahren zur Herstellung einer Verbindung der Formel I

$$R^1-\overset{R^1}{\overset{|}{N}}-\overset{S}{\overset{\|}{C}}-A-B-NH-\overset{R^2}{\overset{|}{C}}H-D-E-X-Y \qquad (I)$$

in welcher

$R^1$ unabhängig voneinander Wasserstoff, $(C_1-C_{10})$-Alkyl, das gegebenenfalls ein- oder zweifach ungesättigt ist und das gegebenenfalls durch bis zu 3 gleiche oder verschiedene Reste aus der Reihe Hydroxy, $(C_1-C_7)$-Alkoxy, $(C_1-C_7)$-Alkanoyloxy- Carboxy, $(C_1-C_7)$-Alkoxycarbonyl, Cl, Br, Amino, $(C_1-C_7)$-Alkylamino, Di-$(C_1-C_7)$-alkylamino, $(C_1-C_5)$-Alkoxycarbonylamino, $(C_7-C_{15})$-Aralkoxycarbonylamino und 9-Fluorenylmethyloxycarbonylamino substituiert ist, $(C_3-C_8)$-Cycloalkyl, $(C_3-C_8)$-Cycloalkyl-$(C_1-C_6)$-alkyl, $(C_6-C_{14})$-Aryl, das gegebenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogen substituiertes Anilino und Trifluormethyl substituiert ist; $(C_6-C_{14})$-Aryl-$(C_1-C_6)$-alkyl, worin der Arylteil gegebenenenfalls durch einen oder zwei gleiche oder verschiedene Reste aus der Reihe F, Cl, Br, J, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1 - 4 N-Atomen und/oder 1 S- oder O-Atom als Ringglieder steht, der gegebenenfalls durch einen, zwei oder drei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist, bedeutet oder zusammen mit dem benachbarten Stickstoffatom einen 3-bis 8 gliedrigen Monocyclus oder einen 7- bis 13-gliedrigen Bicyclus mit mindestens 1 C-Atom, 1 - 4 N-Atomen und/oder 1 S-oder O-Atom als Ringglieder, der gegebenfalls durch ein, zwei oder drei gleiche oder verschiedene Reste aus der Reihe $(C_1-C_6)$-Alkyl, Hydroxy, Hydroxy-$(C_1-C_6)$-alkyl, $(C_1-C_6)$-Alkoxy, $(C_1-C_6)$-Alkoxy-$(C_1-C_6)$-alkyl, Carboxy, Carboxy-$(C_1-C_6)$-alkyl, $(C_1-C_6)$-Alkoxycarbonyl, $(C_1-C_6)$-Alkoxycarbonyl-$(C_1-C_6)$-alkyl, Halogen, Amino, Amino-$(C_1-C_6)$-alkyl, $(C_1-C_6)$-Alkylamino-$(C_1-C_6)$-alkyl, Di-$(C_1-C_6)$-alkylamino oder Di-$(C_1-C_6)$-alkylamino-$(C_1-C_6)$-alkyl substituiert sein kann, bildet;

A einen N-terminal mit -CS- und C-terminal mit B verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, Benzodioxol-5-ylalanin, N-Methyl-histidin, 2-Amino-4-(3-thienyl)buttersäure, 3-(2-Thienyl)-serin, (Z)-Dehydrophenylalanin, (E)-Dehydrophenylalanin, (1,3-Dioxolan-2-yl)alanin, N-Pyrrolylalanin, (1-, 3- oder 4-Pyrazolyl)alanin, (4-Thiazolyl)alanin, (2-, 4- oder 5-Pyrimidyl)alanin, Cyclopentylglycin, tert. Butylglycin, Phenylserin, bedeutet, wobei die Aminogruppe gegebenenfalls jeweils durch ($C_1$-$C_6$)-Alkyl, Formyl, ($C_1$-$C_6$)-Alkoxycarbonyl oder Benzyloxycarbonyl, substituiert sein kann;

B einen N-terminal mit A und C-terminal mit -NH-CH-$R^2$-verknüpften Rest einer natürlichen oder unnatürlichen Aminosäure, wie für A definiert, wobei die Aminogruppe gegebenenfalls wie unter A beschrieben substituiert sein kann;

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl, ($C_6$-$C_{14}$)-Aryl-($C_1$-$C_4$)-alkyl oder Heterocyclyl-($C_1$-$C_4$)-alkyl bedeutet, wobei der Heterocyclus 4 - 7 Ringglieder, davon 1 oder 2 Schwefel- und/oder Sauerstoffatome, enthält;

D eine Gruppe aus der Reihe

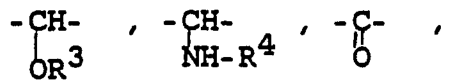

-$CH_2$-$SO_x$- oder -$CH_2$-NH- bedeutet, worin

$R^3$-Wasserstoff; ($C_1$-$C_{10}$)-Alkyl; ($C_1$-$C_6$)-Alkanoyl; ($C_6$-$C_9$)-Cycloalkanoyl; Phenyl, Phenyl-($C_1$-$C_4$)-alkyl, Benzoyl, die jeweils gegebenenfalls im Aromaten durch einen, zwei oder drei Reste aus der Gruppe ($C_1$-$C_4$)-Alkyl, ($C_1$-$C_4$)-Alkoxy, Cl, F, Br, Nitro, Trifluormethyl oder ein Methylendioxy substituiert sind; ($C_1$-$C_{10}$)-Alkylaminothiocarbonyl, ($C_1$-$C_{10}$)-Alkylaminocarbonyl, wobei der ($C_1$-$C_{10}$)-Alkylrest Jeweils wie unter $R^1$ beschrieben substituiert sein kann, ($C_1$-$C_6$)-Alkanoyloxy-($C_1$-$C_2$)-alkyl, ($C_1$-$C_6$)-Alcoxycarbonyloxy-($C_1$-$C_2$)-alkyl oder

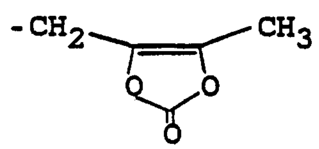

bedeutet,

$R^4$ Wasserstoff, ($C_1$-$C_6$)-Alkanoyl, ($C_5$-$C_8$)-Cycloalkanoyl, Phenylcarbonyl, Phenyl-($C_1$-$C_4$)-alkanoyl, N-($C_1$-$C_4$)-Alkyl-1,4-dihydronicotinoyl, einen Rest

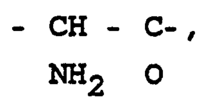

worin R die Seitenkette einer natürlichen Aminosäure ist, ($C_1$-$C_6$)-Alkoxycarbonyl, ($C_5$-$C_8$)-Cycloalkyloxycarbonyl, Phenoxycarbonyl, ($C_1$-$C_6$)-Alkanoyloxy-($C_1$-$C_2$)-alkoxycarbonyl, Phenylcarbonyloxy-($C_1$-$C_2$)-alkoxycarbonyl, ($C_1$-$C_6$)-Alkanoyloxy-($C_1$-$C_2$)-alkyl, Phenylcarbonyloxy-($C_1$-$C_2$)-alkyl, ($C_1$-$C_6$)-Alkanoylaminomethyl, Phenylcarbonylaminomethyl oder ein gegebenenfalls substituiertes, über die 3-Position angeknüpftes Acrylsäurederivat, wobei in allen Resten $R^4$, die Phenyl enthalten, dieser Phenylrest gegebenenfalls durch ein, zwei oder drei Reste aus der Gruppe ($C_1$-$C_4$)-Alkyl, ($C_1$-$C_4$)-Alkoxy, Cl, Br, F, Nitro, Trifluormethyl oder ein Methylendioxy substituiert sein kann, bedeuten

x 0,1 oder 2 bedeutet

und

E abwesend ist oder eine Gruppe aus der Reihe -$(CH_2)_{1-3}$-, -CHF-, -$CF_2$-,

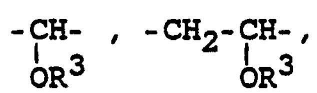

-$CH_2$-$SO_y$-,

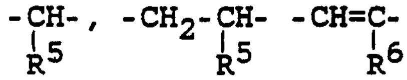

bedeutet,
worin
$R^3$ wie oben definiert ist,
$R^5$ die Seitenkette einer natürlichen Aminosäure, Hydroxy-($C_1$-$C_6$)-alkyl, ($C_1$-$C_6$)-Alkoxy oder ($C_2$-$C_6$)-Alkenyloxy bedeutet,
$R^6$ Wasserstoff oder ($C_1$-$C_6$)-Alkyl ist, y 0,1 oder 2 bedeutet,
und
X abwesend ist oder eine Gruppe aus der Reihe
$-C(=O)-$, $-(CF_2)_{1-4}-$, $-NH-C(=O)-$ bedeutet
und
Y Wasserstoff, Fluor, ($C_1$-$C_6$)-Alkyl, ($C_5$-$C_8$)-Cycloalkyl, ($C_1$-$C_6$)-Alkoxy, ($C_5$-$C_8$)-Cycloalkoxy, Amino, ($C_1$-$C_6$)-Alkylamino, Di-($C_1$-$C_6$)-alkylamino, ($C_6$-$C_{14}$)-Aryl, ($C_6$-$C_{14}$)-Aryl-($C_1$-$C_6$)-alkyl, wobei jeweils der Arylrest unsubstituiert oder, wie oben unter $R^3$ definiert, mono-, di- oder trisubstituiert sein kann; Hydroxy, Azido, Azido-($C_1$-$C_6$)-alkyl, ($C_1$-$C_6$)-Alkylthio, ($C_1$-$C_6$)-Alkylsulfonyl, ($C_5$-$C_8$)-Cycloalkylthio, ($C_5$-$C_8$)-Cycloalkylsulfonyl, Heterocyclyl oder Heterocyclyl-($C_1$-$C_6$)-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist, bedeutet,
oder deren physiologisch verträgliche Salze, dadurch gekennzeichnet, daß man

a) eine Verbindung der allgemeinen Formel II

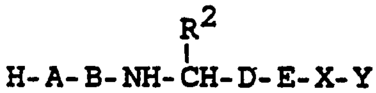

(II)

in welcher A, B, D, E, X, Y und $R^2$ die gleiche Bedeutung wie in der allgemeinen Formel I haben, mit einem Isothiocyanat der allgemeinen Formel III
$R^1$-N=C=S (III)
worin $R^1$ die gleiche Bedeutung wie in der allgemeinen Formel I hat,
in einem geeigneten organischen Lösungsmittel wie z. B. Benzol, Toluol, Chlorbenzol, einem aliphatischen Kohlenwasserstoff, einem aliphatischen Chlorkohlenwasserstoff, Diethylether, Tetrahydrofuran, Dioxan, Aceton, Acetonitril, Dimethylformamid, Dimethylsulfoxid, oder aber unter Verzicht auf ein Lösungsmittel jeweils wahlweise mit oder ohne Zusatz einer Lewis-Säure oder einer Lewis-Base als Katalysator, wie z. B. eines tertiären Amins, vorzugsweise Triethylamin, 1,4-Diazabicyclo[2.2.2]octan, Cyclohexyldimethylamin, Benzyldimethylamin, 4-Methylmorpholin, Tetramethylguanidin oder 4-Dimethylaminopyridin, bei einer Temperatur zwischen -80° C und der Siedetemperatur des Lösungsmittels, vorzugsweise zwischen -30° C und +80° C, umsetzt, gegebenenfalls zuvor empfindliche funktionelle Gruppen wie Hydroxy- oder Aminogruppen mit einer Schutzgruppe versieht und diese Schutzgruppe am Ende wieder entfernt,
oder

b) eine Verbindung der allgemeinen Formel IV mit freier Carboxylgruppe

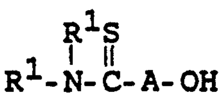

(IV)

worin $R^1$ und A die gleiche Bedeutung wie in Formel I haben, oder ein reaktives Derivat hiervon mit dem entsprechenden Fragment mit einer freien Aminogruppe der allgemeinen Formel V

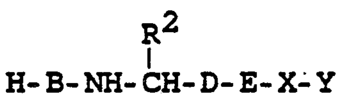

(V)

worin B, D, E, X, Y und $R^2$ die gleiche Bedeutung wie in Formel I besitzen, kuppelt und gegebenenfalls zum Schutz weiterer funktioneller Gruppen (eine) temporär eingeführte Schutzgruppe(n) abspaltet, oder

c) eine Verbindung der allgemeinen Formel VI mit freier Carboxylgruppe

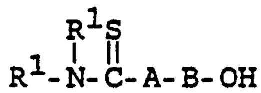

(VI)

worin $R^1$, A und B die gleiche Bedeutung wie in Formel I haben, oder ein reaktives Derivat hiervon mit dem entsprechenden Fragment mit einer freien Aminogruppe der allgemeinen Formel VII

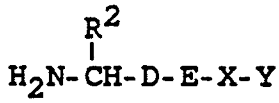

(VII)

worin $R^2$, D, E, X und Y die gleiche Bedeutung wie in Formel I haben, kuppelt und gegebenenfalls zum Schutz weiterer funktioneller Gruppen (eine) temporär eingeführte Schutzgruppe(n) abspaltet, und die so erhaltene Verbindung gegebenenfalls in ihr physiologisch verträgliches Salz überführt.

2. Verfahren der Formel I gemäß Anspruch 1, dadurch gekennzeichnet, daß

$R^1$ unabhängig voneinander Wasserstoff, $(C_1-C_{10})$-Alkyl, das gegebenenfalls bis zu drei gleiche oder verschiedene Reste aus der Reihe Hydroxy, $(C_1-C_4)$-Alkoxy, $(C_1-C_4)$-Alkanoyloxy, Carboxy, $(C_1-C_4)$-Alkoxycarbonyl, Cl, Br, Amino, $(C_1-C_4)$-Alkylamino, Di-$(C_1-C_4)$-alkylamino oder $(C_1-C_4)$-Alkoxycarbonylamino substituiert ist, $(C_5-C_7)$-Cycloalkyl, $(C_6-C_{14})$-Aryl, das gegebenenfalls durch einen oder zwei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino und Trifluormethyl substituiert ist, $(C_6-C_{14})$-Aryl-$(C_1-C_6)$-alkyl, worin der Arylteil gegebenenfalls durch einen oder zwei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino, $(C_1-C_4)$-Alkylamino, Di-$(C_1-C_4)$-alkylamino, Carboxy, Carboxymethoxy, Amino-$(C_1-C_4)$-alkyl, $(C_1-C_4)$-Alkylamino-$(C_1-C_4)$-alkyl, Di-$(C_1-C_4)$-alkylamino-$(C_1-C_4)$-alkyl, $(C_1-C_4)$-Alkoxycarbonylmethoxy, Carbamoyl, Sulfamoyl, $(C_1-C_4)$-Alkoxysulfonyl, Sulfo- und Guanidinomethyl substituiert ist, oder für den Rest eines 3- bis 8-gliedrigen monocyclischen oder 7- bis 13-gliedrigen bicyclischen Heterocyclus mit mindestens 1 C-Atom, 1 - 4 N-Atomen und/oder 1 S- oder O-Atomen als Ringglieder steht, der gegebenenfalls durch einen, zwei oder drei gleiche oder verschiedene Reste aus der Reihe F, Cl, Br, Hydroxy, $(C_1-C_4)$-Alkoxy, $(C_1-C_4)$-Alkyl, $(C_1-C_4)$-Alkoxycarbonyl, Amino oder Trifluormethyl substituiert ist, bedeutet oder zusammen mit dem benachbarten Stickstoffatom und $R^1$ einen 3- bis 8-gliedrigen Monocyclus oder einen 7- bis 13-gliedrigen Bicyclus mit mindestens 1 C-Atom, 1 - 4 N-Atomen und/oder 1 S- oder O-Atom als Ringglieder, der gegebenfalls durch ein, zwei oder drei gleiche oder verschiedene Reste aus der Reihe $(C_1-C_6)$-Alkyl, Hydroxy, Hydroxy-$(C_1-C_6)$-alkyl, $(C_1-C_6)$-Alkoxy, $(C_1-C_6)$-Alkoxy-$(C_1-C_6)$-alkyl, Carboxy, Carboxy-$(C_1-C_6)$-alkyl, $(C_1-C_6)$-Alkoxycarbonyl, $(C_1-C_6)$-Alkoxycarbonyl-$(C_1-C_6)$-alkyl, Halogen, Amino, Amino-$(C_1-C_6)$-alkyl, $(C_1-C_6)$-Alkylamino-$(C_1-C_6)$-alkyl, Di-$(C_1-C_6)$-alkylamino oder Di-$(C_1-C_6)$-alkylamino-$(C_1-C_6)$-alkyl substituiert sein kann, bildet;

A einen N-terminal mit -CS- und C-terminal mit B verknüpften Rest einer natürlichen oder unnatürlichen Aminosäure aus der Reihe Phenylalanin, Histidin, Tyrosin, Tryptophan, ß-2-Thienylalanin, ß-3-Thienylalanin, ß-2-Furylalanin, ß-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyldopa, 2-Amino-4-(2-thienyl)-buttersäure, Benzodioxol-5-ylalanin, N-Methyl-histidin, 2-Amino-4-(3-thienyl)buttersäure, 3-(2-Thienyl)-serin,(Z)-Dehydrophenylalanin oder (E)-Dehydrophenylalanin, bedeutet;

B einen N-terminal mit A und C-terminal mit -NH-CH-$R^2$-verknüpften Rest einer natürlichen oder unnatürlichen Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, Benzodioxol-5-ylalanin, N-Methyl-histidin, 2-Amino-4-(3-thienyl)-buttersäure, 3-(2-Thienyl)-serin,(Z)-Dehydrophenylalanin, (E)-Dehydrophenylalanin, (1,3-Dioxolan-2-yl)alanin, N-Pyrrolylalanin, (1-, 3- oder 4-Pyrazolyl)alanin, (4-Thiazolyl)alanin, (2-, 4- oder 5-Pyrimidyl)alanin, Cyclopentylglycin, tert. Butylglycin oder Phenylserin, bedeutet,

$R^2$ Wasserstoff, $(C_1-C_{10})$-Alkyl, $(C_4-C_7)$-Cycloalkyl, $(C_4-C_7)$-Cycloalkyl-$(C_1-C_4)$-alkyl, $(C_6-C_{14})$-Aryl, $(C_6-C_{14})$-Aryl-$(C_1-C_4)$-alkyl oder Heterocyclyl-$(C_1-C_4)$-alkyl bedeutet, wobei der Heterocyclus 4 - 7 Ringglieder, davon 1 oder 2 Schwefel-und/oder Sauerstoffatome, besitzt,

und

D eine Gruppe aus der Reihe

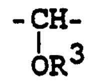 , 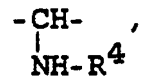 , 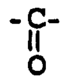 ,

$-CH_2-SO_x-$ oder $-CH_2-NH-$bedeutet, worin

$R^3$ Wasserstoff,

$R^4$ Wasserstoff,

x 0, 1 oder 2 bedeutet

und

E abwesend ist oder eine Gruppe aus der Reihe $-(CH_2)_{1-3}-$, -CHF-, $-CF_2-$,

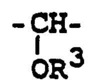 , 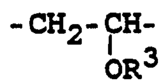 ,

$-CH_2-SO_y-$,

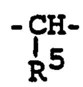 , 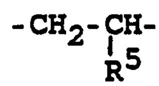 , 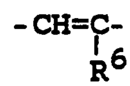

bedeutet

worin

$R^3$ Wasserstoff ist,

$R^5$ die Seitenkette einer natürlichen Aminosäure, Hydroxy-$(C_1-C_6)$-alkyl, $(C_1-C_6)$-Alkoxy oder $(C_2-C_6)$-Alkenyloxy bedeutet,

$R^6$ Wasserstoff oder $(C_1-C_6)$-Alkyl ist,

y 0, 1 oder 2 bedeutet

und

X abwesend ist oder eine Gruppe aus der Reihe

$-\underset{\underset{O}{\|}}{C}-$, $-(CF_2)_{1-4}-$, $-NH-\underset{\underset{O}{\|}}{C}-$ bedeutet

und

Y Wasserstoff, Fluor, $(C_1-C_6)$-Alkyl, $(C_5-C_8)$-Cycloalkyl, $(C_1-C_6)$-Alkoxy, $(C_5-C_8)$-Cycloalkoxy, Amino, $(C_1-C_6)$-Alkylamino, Di-$(C_1-C_6)$-alkylamino, $(C_6-C_{14})$-Aryl, $(C_6-C_{14})$-Aryl-$(C_1-C_6)$-alkyl, wobei jeweils der Arylrest unsubstituiert oder, wie oben unter $R^3$ definiert, mono-, di oder trisubstituiert sein kann, Hydroxy, Azido, Azido-$(C_1-C_6)$-alkyl, $(C_1-C_6)$-Alkylthio, $(C_1-C_6)$-Alkylsulfonyl, $(C_5-C_8)$-Cycloalkylthio, $(C_5-C_8)$-Cycloalkylsulfonyl, Heterocyclyl oder Heterocyclyl-$(C_1-C_6)$-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist bedeutet.

3. Verfahren gemäß einem oder mehreren der Ansprüche 1 bis 2, dadurch gekennzeichnet, daß

$R^1$ unabhängig voneinander Wasserstoff, $(C_1-C_{10})$-Alkyl, das gegebenenfalls durch bis zu drei gleiche oder verschiedene Reste aus der Reihe Hydroxy, Carboxy, Amino, $(C_1-C_4)$-Alkylamino, Di$(C_1-C_4)$-alkylamino oder $(C_1-C_4)$-Alkoxycarbonylamino substituiert ist, Phenyl, das gegebenenfalls durch ein oder zwei Reste aus der Reihe F, Cl, Hydroxy, Amino oder Trifluormethyl substituiert ist, Phenyl-$(C_1-C_4)$-alkyl, worin der Phenylteil gegebenenfalls durch einen oder zwei gleiche oder verschiedene Reste aus der Reihe F, Cl,

Hydroxy, Amino, $(C_1-C_4)$-Alkylamino, Di-$(C_1-C_4)$-alkylamino, Carboxy, Amino-$(C_1-C_4)$-alkyl, $(C_1-C_4)$-Alkylamino-$(C_1-C_4)$-alkyl, Di-$(C_1-C_4)$-alkylamino-$(C_1-C_4)$-alkyl substituiert ist, bedeutet, oder zusammen mit dem benachbarten Stickstoffatom und $R^1$ einen 3- bis 8-gliedrigen Monocyclus oder einen 7- bis 13-gliedrigen Bicyclus mit mindestens 1 C-Atom, 1-4 N-Atomen und/oder 1 S- oder O-Atom als Ringglieder, der gegebenenfalls durch ein, zwei oder drei gleiche oder verschiedene Reste aus der Reihe $(C_1-C_6)$-Alkyl, Hydroxy, Hydroxy-$(C_1-C_6)$-alkyl, $(C_1-C_6)$-Alkoxy-$(C_1-C_6)$-alkyl, Carboxy, Carboxy-$(C_1-C_6)$-alkyl, $(C_1-C_6)$-Alkoxycarbonyl, $(C_1-C_6)$-Alkoxycarbonyl-$(C_1-C_6)$-alkyl, Halogen, Amino, Amino-$(C_1-C_6)$-alkyl, $(C_1-C_6)$-Alkylamino-$(C_1-C_6)$-alkyl, Di-$(C_1-C_6)$alkylamino oder Di-$(C_1-C_6)$-alkylamino-$(C_1-C_6)$-alkyl substituiert sein kann, bildet

A einen N-terminal mit -CS- und C-terminal mit B verknüpften Rest einer Aminosäure aus der Reihe Phenylalanin, Tyrosin, ß-2-Thienylalanin, ß-3-Thienylalanin, 4-Chlorphenylalanin, O-Methyltyrosin, 1-Naphthylalanin, 2-Fluorphenylalanin, 3-Fluorphenylalanin, 4-Fluorphenylalanin bedeutet,

B einen N-terminal mit A und C-terminal mit -NH-CHR$^2$-verknüpften Rest einer Aminosäure aus der Reihe Phenylalanin, Histidin, Leucin, ß-2-Thienylalanin, ß-3-Thienylalanin, Lysin, Norvalin, 2-Fluorphenylalanin, 3-Fluorphenylalanin, 4-Fluorphenylalanin, Norleucin, S-Methylcystein, (1,3-Dioxolan-2-yl)alanin, (1-, 3- oder 4-Pyrazolyl)alanin, 4-Thiazolylalanin, (2-, 4- oder 5-Pyrimidyl)alanin, bedeutet

$R^2$ Isobutyl, Cyclohexylmethyl, Benzyl oder (1,3-Dithiolan-2-yl)methyl bedeutet,

D eine Gruppe

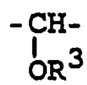

bedeutet und $R^3$ Wasserstoff ist,

E abwesend ist oder eine Gruppe aus der Reihe $-(CH_2)_{1-3}-$,

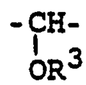 , 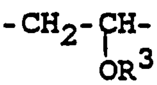 , 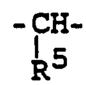

bedeutet,

worin

$R^3$ Wasserstoff ist und

$R^5$ die Seitenkette einer natürlichen Aminosäure bedeutet

X abwesend ist oder eine Gruppe aus der Reihe

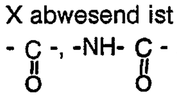 bedeutet

und

Y Wasserstoff, $(C_1-C_6)$-Alkyl, $(C_5-C_8)$-Cycloalkyl, Amino, $(C_1-C_6)$-Alkylamino, Di-$(C_1-C_6)$-alkylamino, $(C_6-C_{14})$-Aryl, $(C_6-C_{14})$-Aryl-$(C_1-C_6)$-alkyl, wobei jeweils der Arylrest unsubstituiert oder, wie oben unter $R^3$ definiert, mono-, di- oder trisubstituiert sein kann, Hydroxy, Azido, $(C_1-C_6)$-Alkylthio, $(C_1-C_6)$-Alkylsulfonyl, $(C_5-C_8)$-Cycloalkylthio, $(C_5-C_8)$-Cycloalkylsulfonyl, Heterocyclyl oder Heterocyclyl-$(C_1-C_6)$-alkyl, wobei der Heterocyclus jeweils ein 3- bis 8-gliedriger Monocyclus oder ein 7- bis 13-gliedriger Bicyclus mit mindestens einem C-Atom und einem bis sechs Heteroatomen aus der Reihe O, N oder S ist bedeutet.

4. Verwendung einer nach einem Verfahren der Ansprüche 1 bis 3 hergestellten Verbindung der Formel I als Heilmittel.

5. Verwendung einer nach einem Verfahren der Ansprüche 1 bis 3 hergestellten Verbindung der Formel I zur Behandlung des Bluthochdrucks.

6. Verfahren zur Herstellung einer pharmazeutischen Zubereitung enthaltend eine nach einem der Verfahren der Ansprüche 1 bis 3 hergestellten Verbindung der Formel I dadurch gekennzeichnet, daß man diese zusammen mit einem physiologisch unbedenklichen Träger und gegebenenfalls weiteren Hilfs- und Zusatzstoffen in eine geeignete Darreichungsform bringt.